# EUROPÄISCHE PATENTSCHRIFT

(11) **EP 3 131 887 B1**
(45) Veröffentlichungstag und Bekanntmachung des Hinweises auf die Patenterteilung: **02.09.2020**
(21) Anmeldenummer: 15713794.4
(22) Anmeldetag: 08.04.2015
(51) Int. Cl.: C07D 309/10

(54) **HERSTELLUNG VON 2-SUBSTITUIERTEN 4-HYDROXY-4-METHYL-TETRAHYDROPYRANEN MIT STABILER RIECHSTOFFQUALITÄT**
PRODUCTION OF 2-SUBSTITUTED 4-HYDROXY-4-METHYL-TETRAHYDROPYRANES WITH STABLE ODOUR QUALITY
FABRICATION DE 4-HYDROXY-4-MÉTHYL-TETRAHYDROPYRANES 2-SUBSTITUÉS AYANT UNE QUALITÉ DE PARFUM STABLE

(30) Priorität: 14.04.2014 EP 14164594
(43) Veröffentlichungstag der Anmeldung: 22.02.2017
(73) Patentinhaber: BASF SE, 67056 Ludwigshafen am Rhein (DE)
(72) Erfinder: STORK, Timon, 68642 Bürstadt Bobstadt (DE); DEHN, Martine, 67061 Ludwigshafen (DE); EBEL,Klaus, 68542 Heddesheim (DE); BECK, Karl, 76684 Östringen (DE); SALDEN, Axel, 70193 Stuttgart (DE); PELZER, Ralf, 37699 Fürstenberg (DE)
(86) Internationale Anmeldenummer: PCT/EP2015/057584
(87) Internationale Veröffentlichungsnummer: WO 2015/158586

(56) Entgegenhaltungen:
- WO-A1-2010/133473
- US-A1- 2011 306 779

## Beschreibung

### HINTERGRUND DER ERFINDUNG

Die vorliegende Erfindung betrifft ein Verfahren zur Herstellung von 2-substituierten 4-Hydroxy-4-methyl-tetrahydropyranen aus der sauer katalysierten Umsetzung von 3-Methylbut-3-en-1-ol mit einem Aldehyd, wobei eine stabile Riechstoffqualität ohne den Geruchseindruck störende Fehlnoten erzielt wird.

### STAND DER TECHNIK

2-substituierte 4-Hydroxy-4-methyl-tetrahydropyrane sind wertvolle Verbindungen für den Einsatz als Aromachemikalien. So zeichnet sich beispielsweise das cis/trans-Diastereomerengemisch des 2-(2-Methylpropyl)-4-hydroxy-4-methyl-tetrahydropyrans 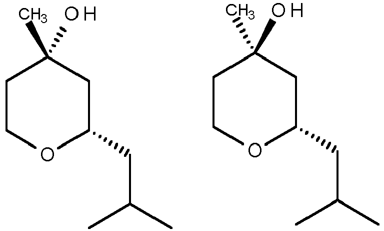 durch einen angenehmen Maiglöckchenduft aus und ist in besonderem Maße zur Verwendung als Aromachemikalie, z. B. zur Herstellung von Riechstoffkompositionen, geeignet.

Die EP 1 493 737 A1 offenbart ein Verfahren zur Herstellung von Gemischen von ethylenisch ungesättigten 4-Methyl- bzw. 4-Methylenpyranen und den entsprechenden 4-Hydroxypyranen durch Umsetzung der entsprechenden Aldehyde mit Isoprenol, wobei die Umsetzung in einem Reaktionssystem initiiert wird, in dem das molare Verhältnis von Aldehyd zu Isoprenol größer als 1 ist, d. h. der Aldehyd im Überschuss eingesetzt wird. Darüber hinaus offenbart das Dokument die anschließende Dehydratisierung der genannten Gemische zu den gewünschten ethylenisch ungesättigten Pyranen.

Die WO 2011/147919 beschreibt ein Verfahren zur Herstellung von 2-substituierten 4-Hydroxy-4-methyl-tetrahydropyranolen und speziell von 2-Isobutyl-4-hydroxy-4-methyl-tetrahydropyran durch Umsetzung von Isoprenol mit Prenal und anschließender Hydrierung.

Die WO 2010/133473 beschreibt ein Verfahren zur Herstellung von 2-substituierten 4-Hydroxy-4-methyl-tetrahydropyranen der Formel (A) 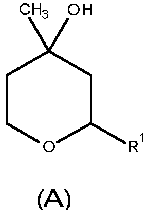 wobei der Rest R¹ für einen geradkettigen oder verzweigten Alkyl- oder Alkenylrest mit 1 bis 12 Kohlenstoffatomen, einen gegebenenfalls Alkyl-substituierten Cycloalkylrest mit insgesamt 3 bis 12 Kohlenstoffatomen oder einen gegebenenfalls Alkyl- und/oder Alkoxy-substituierten Arylrest mit insgesamt 6 bis 12 Kohlenstoffatomen steht, bei dem man Isoprenol (3-Methylbut-3-en-1-ol) mit einem Aldehyd der Formel R¹-CHO umsetzt, wobei man die Umsetzung in Gegenwart von Wasser und in Gegenwart eines stark sauren Kationenaustauschers durchführt.

Die WO 2011/154330 beschreibt ein zur WO 2010/133473 vergleichbares Verfahren, wobei das erhaltene Reaktionsgemisch einer destillativen Aufarbeitung in einer Trennwandkolonne oder in zwei thermisch gekoppelten Destillationskolonnen unterzogen wird.

Wie in der WO 2010/133473 und WO 2011/154330 beschrieben ist, fällt bei der sauer katalysierten Umsetzung von Isoprenol (3-Methylbut-3-en-1-ol) mit einem Aldehyd der Formel R¹-CHO ein komplexes Reaktionsgemisch an, das neben 2-substituierten 4-Hydroxy-4-methyltetrahydropyranen noch dehydratisierte Nebenprodukte der Formeln (D), (E) und/oder (F) 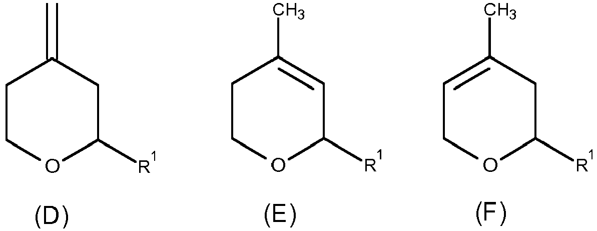 sowie als weitere Nebenprodukte unter anderem die 1,3-Dioxane (G) 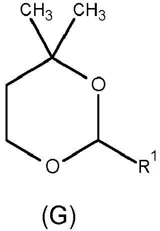 enthält.

Die unveröffentlichte internationale Patentanmeldung PCT/EP2013/071409 beschreibt ein Verfahren zur Herstellung von 2-substituierten 4-Hydroxy-4-methyl-tetrahydropyranen der allgemeinen Formel (A) und von 2-substituierten 4-Methyl-tetrahydropyranen der allgemeinen Formel (B) 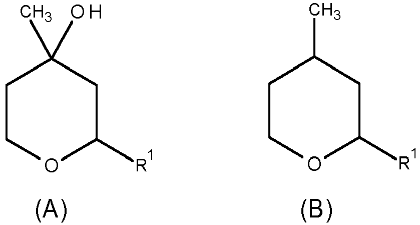 worin
- R¹: für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
bei dem man
a) 3-Methylbut-3-en-1-ol mit einem Aldehyd der Formel R¹-CHO, wobei R¹ in der Formel die zuvor angegebene Bedeutung hat, in Gegenwart eines sauren Katalysators umsetzt, wobei ein Reaktionsgemisch erhalten wird, das wenigstens ein 2-substituiertes 4-Hydroxy-4-methyl-tetrahydropyran der allgemeinen Formel (A), wenigstens eine der Verbindungen (D), (E) oder (F) und wenigstens eine Dioxanverbindung (G) enthält 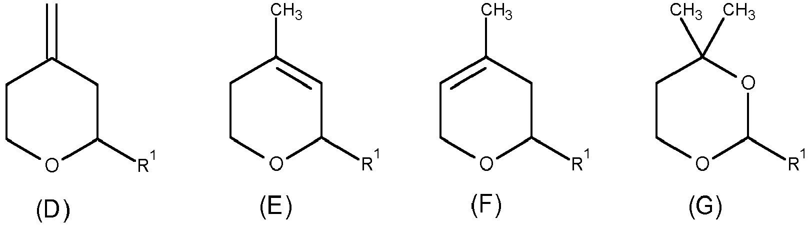 wobei R¹ die zuvor angegebene Bedeutung hat,
b) das Reaktionsprodukt aus Schritt a) einer Auftrennung unter Erhalt einer an 2-substituierten 4-Hydroxy-4-methyl-tetrahydropyranen der allgemeinen Formel (A) angereicherten Fraktion und einer Fraktion, die wenigstens eine der Verbindungen (D), (E) oder (F) und wenigstens eine Dioxanverbindung (G) enthält, unterzieht,
c) die Fraktion, die wenigstens eine der Verbindungen (D), (E) oder (F) und wenigstens eine Dioxanverbindung (G) enthält, einer Hydrierung unterzieht,
d) aus dem in Schritt c) erhaltenen Hydrierprodukt eine an 2-substituierten 4-Methyl-tetrahydropyranen (B) angereicherte Fraktion und eine an der wenigstens einen Dioxanverbindung (G) angereicherte Fraktion isoliert.

Romanov et al. beschreiben im Journal of Applied Chem. of the USSR, 55 (1), S. 140 - 143 (1982) (englische Übersetzung aus Zhurnal Prikladnoi Khimii, Bd. 55, Nr. 1, 157 - 161 (1981)) die sauer katalysierte Umsetzung der Dioxanverbindung G') zu den Dihydropyranen E') und F').

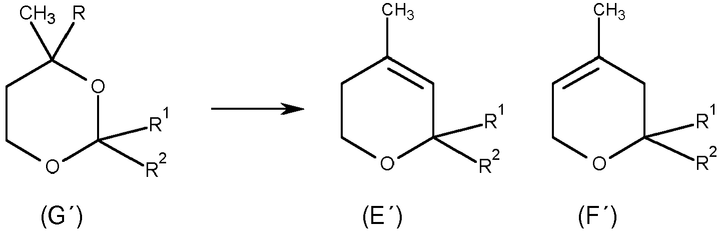

Tabellarisch erwähnt sind 4-Methyl-2-isobutyl-5,6-dihydropyran und 4-Methyl-2-isobutyl-3,6-dihydropyran. Als saure Katalysatoren werden H₂SO₄ oder Sulfonsäuregruppen-haltige Styrol-Divinylbenzol-Ionenaustauscher eingesetzt. Die Reaktion erfolgt mit Dioxanverbindungen G') in Reinform und in Gegenwart von Cyclohexan oder Toluol als Lösungsmittel.

Romanov et al. beschreiben im Journal of Applied Chem. of the USSR, 56 (1), S. 2526 - 2528 (1983) (englische Übersetzung aus Zhurnal Prikladnoi Khimii, Bd. 55, Nr. 12, 2778 - 2780 (1982)) die sauer katalysierte Isomerisierung von 2-R-4,4-Dimethyl- und 2-R-4-Methyl-4-phenyl-1,3-dioxanen zu 2-R-4,4-Methyl- und 2-R-4-Phenyl-1,3-tetrahydropyran-4-olen.

Eine wesentliche Anforderung an die Synthese von Aromachemikalien und speziell von Geruchsstoffen ist die gleichbleibend hohe Qualität des Produkts. Ein wesentliches Qualitätskriterium ist, dass eine stabile Riechstoffqualität ohne den Geruchseindruck störender Fehlnoten erzielt wird. Dabei dürfen weder bei der Herstellung im Batch-Verfahren einzelne Chargen unerwünschte Geruchsnoten aufweisen noch darf sich bei der kontinuierlichen Herstellung die Riechstoffqualität im Verlauf der Herstellung verschlechtern. Letzteres kann beispielsweise auf Alterungsprozesse des eingesetzten Katalysators und damit verbundene Änderungen im Produktspektrum zurückzuführen sein. Speziell bei der Herstellung von 2-substituierten 4-Hydroxy-4-methyl-tetrahydropyranen aus der sauer katalysierten Umsetzung von 3-Methylbut-3-en-1-ol mit einem Aldehyd wird manchmal beobachtet, dass die Riechstoffqualität, speziell des formulierten Endprodukts nach längerer Lagerung, nicht mehr der Erwartung entspricht. In diesen Fällen weist das Produkt häufig eine unerwünschte käsige Geruchsnote auf. Der vorliegenden Erfindung liegt die Aufgabe zugrunde, ein verbessertes Verfahren zur Herstellung von 2-substituierten 4-Hydroxy-4-methyl-tetrahydropyranen zur Verfügung zu stellen, das dieses Problem vermindert oder vermeidet.

Überraschenderweise wurde jetzt gefunden, dass diese Aufgabe gelöst wird, wenn man das bei der sauer katalysierten Umsetzung von 3-Methylbut-3-en-1-ol mit einem Aldehyd zur Herstellung von 2-substituierten 4-Hydroxy-4-methyl-tetrahydropyranen erhaltene Reaktionsprodukt vor seiner destillativen Auftrennung mit einem sauren Ionenaustauscher in Kontakt bringt und/oder mit einer starken Säure versetzt. Durch diese Säurebehandlung kann die Bildung von störenden Geruchsnoten zuverlässig vermieden werden. Dies ist insbesondere überraschend, als die erfindungsgemäße Herstellung der 2-substituierten 4-Hydroxy-4-methyl-tetrahydropyrane bereits in Gegenwart eines sauren Katalysators erfolgt.

### ZUSAMMENFASSUNG DER ERFINDUNG

Gegenstand der Erfindung ist ein Verfahren zur Herstellung von 2-substituierten 4-Hydroxy-4-methyl-tetrahydropyranen der allgemeinen Formel (I) 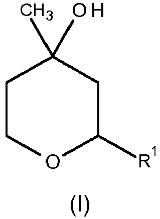 worin
- R¹: für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
bei dem man
a) 3-Methylbut-3-en-1-ol der Formel (IV) 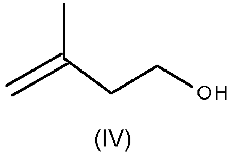 mit einem Aldehyd der Formel (V)

   R¹-CHO (V)

   worin
   - R¹: für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
   in Gegenwart eines sauren Katalysators umsetzt, wobei ein Reaktionsgemisch erhalten wird, das wenigstens ein 2-substituiertes 4-Hydroxy-4-methyl-tetrahydropyran der allgemeinen Formel (I) enthält,
b) das Reaktionsgemisch aus Schritt a) einer destillativen Auftrennung unter Erhalt wenigstens einer an den 2-substituierten 4-Hydroxy-4-methyl-tetrahydropyranen der allgemeinen Formel (I) angereicherten Fraktion unterzieht,
wobei man das Reaktionsgemisch aus Schritt a) vor dem Einsatz in Schritt b) und/oder während des Einsatzes in Schritt b) mit einem sauren Ionenaustauscher in Kontakt bringt und/oder mit einer starken Säure versetzt.

Ausserdem werden 2-substituierte 4-Hydroxy-4-methyl-tetrahydropyrane der allgemeinen Formel (I) beschrieben, die durch das zuvor und im Folgenden definierte Verfahren erhältlich sind. Dabei handelt es sich insbesondere um 2-Isobutyl-4-hydroxy-4-methyl-tetrahydropyran.

Die nach dem erfindungsgemäßen Verfahren erhaltenen 2-substituierten 4-Hydroxy-4-methyl-tetrahydropyrane der allgemeinen Formel (I), insbesondere das 2-Isobutyl-4-hydroxy-4-methyl-tetrahydropyran, eignen sich vorteilhaft für die Verwendung als Aromachemikalie, speziell als Duftstoff.

### BESCHREIBUNG DER ERFINDUNG

Sofern im Folgenden nicht genauer angegeben, bezeichnen die Begriffe "2-substituiertes 4-Hydroxy-4-methyl-tetrahydropyran" und "2-(2-Methylpropyl)-4-hydroxy-4-methyl-tetrahydropyran" im Rahmen der Erfindung cis/trans-Gemische jedweder Zusammensetzung sowie die reinen Konformations-Isomere. Die zuvor genannten Begriffe bezeichnen weiterhin alle Enantiomere in Reinform sowie racemische und optisch aktive Gemische der Enantiomeren dieser Verbindungen.

Sofern im Folgenden von cis- und trans-Diastereomeren der Verbindungen (I) die Rede ist, wird jeweils nur eine der enantiomeren Formen abgebildet. Lediglich zur Veranschaulichung werden im Folgenden die Isomeren des 2-(2-Methylpropyl)-4-hydroxy-4-methyl-tetrahydropyrans (I) wiedergegeben:

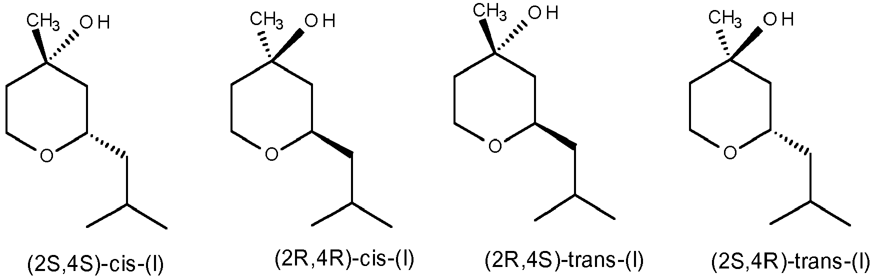

Im Rahmen der vorliegenden Erfindung steht der Ausdruck geradkettiges oder verzweigtes Alkyl vorzugsweise für C₁-C₆-Alkyl und besonders bevorzugt für C₁-C₄-Alkyl. Alkyl steht insbesondere für Methyl, Ethyl, n-Propyl, Isopropyl, n-Butyl, Isobutyl (2-Methylpropyl), sec.-Butyl (1-Methylpropyl), tert.-Butyl (1,1-Dimethylethyl), n-Pentyl oder n-Hexyl. Speziell steht Alkyl für Methyl, Ethyl, n-Propyl, Isopropyl, oder Isobutyl.

Im Rahmen der vorliegenden Erfindung steht der Ausdruck geradkettiges oder verzweigtes Alkoxy vorzugsweise für C₁-C₆-Alkoxy und besonders bevorzugt für C₁-C₄-Alkoxy. Alkoxy steht insbesondere für Methoxy, Ethoxy, n-Propyloxy, Isopropyloxy, n-Butyloxy, Isobutyloxy, sec.-Butyloxy, tert.-Butyloxy, n-Pentyloxy oder n-Hexyloxy. Speziell steht Alkoxy für Methoxy, Ethoxy, n-Propyloxy, Isopropyloxy, oder Isobutyloxy.

Im Rahmen der vorliegenden Erfindung steht der Ausdruck geradkettiges oder verzweigtes Alkenyl vorzugsweise für C₂-C₆-Alkenyl und besonders bevorzugt für C₂-C₄-Alkenyl. Der Alkenylrest weist neben Einfachbindungen noch eine oder mehrere, bevorzugt 1 bis 3, besonders bevorzugt 1 oder 2 und ganz besonders bevorzugt eine ethylenische Doppelbindung auf. Alkenyl steht insbesondere für Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl oder 2-Methyl-2-propenyl.

Im Rahmen der Erfindung bezeichnet Cycloalkyl einen cycloaliphatischen Rest mit vorzugsweise 3 bis 10, besonders bevorzugt 5 bis 8, Kohlenstoffatomen. Beispiele für Cycloalkylgruppen sind insbesondere Cyclopropyl, Cyclobutyl, Cyclopentyl, Cyclohexyl, Cycloheptyl oder Cyclooctyl. Speziell steht Cycloalkyl für Cyclohexyl.

Substituierte Cycloalkylgruppen können in Abhängigkeit von der Ringgröße einen oder mehrere (z. B. 1, 2, 3, 4 oder 5) Substituenten aufweisen. Diese sind vorzugsweise unabhängig voneinander ausgewählt unter C₁-C₆-Alkyl und C₁-C₆-Alkoxy. Die Cycloalkylgruppen tragen im Falle einer Substitution vorzugsweise eine oder mehrere, beispielsweise eine, zwei, drei, vier oder fünf C₁-C₆-Alkylgruppen. Beispiele für substituierte Cycloalkylgruppen sind insbesondere 2- und 3-Methylcyclopentyl, 2- und 3-Ethylcyclopentyl, 2-, 3- und 4-Methylcyclohexyl, 2-, 3- und 4-Ethylcyclohexyl, 2-, 3- und 4-Propylcyclohexyl, 2-, 3- und 4-Isopropylcyclohexyl, 2-, 3- und 4-Butylcyclohexyl und 2-, 3- und 4-Isobutylcyclohexyl.

Der Ausdruck "Aryl" umfasst im Rahmen der vorliegenden Erfindung ein- oder mehrkernige aromatische Kohlenwasserstoffreste mit üblicherweise 6 bis 18, vorzugsweise 6 bis 14, besonders bevorzugt 6 bis 10 Kohlenstoffatomen. Beispiele für Aryl sind insbesondere Phenyl, Naphthyl, Indenyl, Fluorenyl, Anthracenyl, Phenanthrenyl, Naphthacenyl, Chrysenyl, Pyrenyl, etc., und speziell Phenyl oder Naphthyl.

Substituierte Aryle können in Abhängigkeit von der Anzahl und Größe ihrer Ringsysteme einen oder mehrere (z. B. 1, 2, 3, 4 oder 5) Substituenten aufweisen. Diese sind vorzugsweise unabhängig voneinander ausgewählt unter C₁-C₆-Alkyl und C₁-C₆-Alkoxy. Beispiele für substituierte Arylreste sind 2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6-Dipropylphenyl, 2,4,6-Tripropylphenyl, 2-, 3- und 4-lsopropylphenyl, 2,4-, 2,5-, 3,5- und 2,6-Diisopropylphenyl, 2,4,6-Triisopropylphenyl, 2-, 3- und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3- und 4-sec-Butylphenyl, 2,4-, 2,5-, 3,5- und 2,6-Di-sec-butylphenyl, 2,4,6-Tri-sec-butylphenyl, 2-, 3- und 4-tert.-Butylphenyl, 2,4-, 2,5-, 3,5- und 2,6-Di-tert.-butylphenyl und 2,4,6-Tri-tert.-butylphenyl.

Bevorzugt steht R¹ in den Verbindungen der Formeln (I), (II), (III.1), (III.2), (III.3), (V), und (VI) für geradkettiges oder verzweigtes C₁-C₁₂-Alkyl oder geradkettiges oder verzweigtes C₂-C₁₂-Alkenyl. Besonders bevorzugt steht R¹ für geradkettiges oder verzweigtes C₁-C₆-Alkyl oder geradkettiges oder verzweigtes C₂-C₆-Alkenyl. In einer weiteren bevorzugten Ausführung steht R¹ für Phenyl.

Erfindungsgemäß bevorzugte Bedeutungen für den Rest R¹ sind somit beispielsweise Methyl, Ethyl, n-Propyl, Isopropyl, n-Butyl, Isobutyl, n-Pentyl, n-Hexyl oder n-Heptyl, bevorzugt Methyl, Ethyl, n-Propyl, Isopropyl, n-Butyl, Isobutyl, ganz besonders bevorzugt Isobutyl (2-Methylpropyl).

Die vorliegende Erfindung betrifft somit im Rahmen einer bevorzugten Ausführungsform ein Verfahren zur Herstellung und Isolierung von 2-(2-Methylpropyl)-4-hydroxy-4-methyl-tetrahydropyran der Formel (Ia).

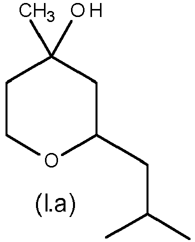

### Schritt a)

Einer der Ausgangsstoffe für Schritt a) des erfindungsgemäßen Verfahrens ist 3-Methylbut-3-en-1-ol (Isoprenol) der Formel (IV),

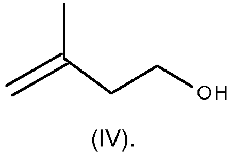

Isoprenol ist nach bekannten Verfahren aus Isobuten und Formaldehyd in jedem Maßstab gut zugänglich und kommerziell verfügbar. An die Reinheit, Qualität oder Herstellverfahren des erfindungsgemäß einzusetzenden Isoprenols sind keine besonderen Anforderungen zu stellen. Es kann in handelsüblicher Qualität und Reinheit in Schritt a) des erfindungsgemäßen Verfahrens eingesetzt werden. Bevorzugt setzt man Isoprenol ein, das eine Reinheit von 90 Gew.-% oder darüber hat, besonders bevorzugt solches mit einer Reinheit von 95 bis 100 Gew.-% und ganz besonders bevorzugt solches mit einer Reinheit von 97 bis 99,9 Gew.-% oder noch mehr bevorzugt 98 bis 99,8 Gew.-%.

Ein weiterer Ausgangsstoff für Schritt a) des erfindungsgemäßen Verfahrens ist ein Aldehyd der Formel (V) R¹-CHO, wobei R¹ in der Formel (V) die zuvor angegebene Bedeutung hat.

Bevorzugt steht R¹ in den Verbindungen der Formel (V) für geradkettiges oder verzweigtes C₁-C₁₂-Alkyl oder geradkettiges oder verzweigtes C₂-C₁₂-Alkenyl. Besonders bevorzugt steht R¹ für geradkettiges oder verzweigtes C₁-C₆-Alkyl oder geradkettiges oder verzweigtes C₂-C₆-Alkenyl. In einer weiteren bevorzugten Ausführung steht R¹ für Phenyl.

Erfindungsgemäß bevorzugte Bedeutungen für den Rest R¹ sind somit beispielsweise Methyl, Ethyl, n-Propyl, Isopropyl, n-Butyl, Isobutyl, n-Pentyl, n-Hexyl oder n-Heptyl, bevorzugt Methyl, Ethyl, n-Propyl, Isopropyl, n-Butyl, Isobutyl, ganz besonders bevorzugt Isobutyl (2-Methylpropyl).

Bevorzugt einzusetzende Aldehyde der Formel (V) sind: Acetaldehyd, Valeraldehyd, Isovaleraldehyd, Pentanal, Hexanal, Heptanal, Benzaldehyd, Citral, Citronellal. Erfindungsgemäß ganz besonders bevorzugt einzusetzende Aldehyde der Formel (V) sind Isovaleraldehyd und Benzaldehyd, insbesondere Isovaleraldehyd.

Bevorzugt werden in Schritt a) das 3-Methylbut-3-en-ol (IV) und der Aldehyd (V) in einem molaren Verhältnis von etwa 1 zu 2 bis 2 zu 1, besonders bevorzugt von 0,7 zu 1 bis 2 zu 1, insbesondere von 1 zu 1 bis 2 zu 1, eingesetzt. In einer speziellen Ausführung werden in Schritt a) das 3-Methylbut-3-en-ol (III) und der Aldehyd (V) in einem molaren Verhältnis von 1 zu 1 bis 1,5 zu 1 eingesetzt.

Erfindungsgemäß erfolgt die Umsetzung in Schritt a) in Gegenwart eines sauren Katalysators. Prinzipiell kann für die Umsetzung in Schritt a) jeder saure Katalysator verwendet werden, d. h. jede Substanz, die Brönstedt- oder Lewis-Acidität aufweist. Beispiele für geeignete Katalysatoren sind Protonensäuren, wie Salzsäure, Schwefelsäure, Phosphorsäure, Methansulfonsäure und p-Toluolsulfonsäure, saure molekulare Elementverbindungen, wie Aluminiumchlorid, Bortrifluorid, Zinkchlorid, Phosphorpentafluorid, Arsentrifluorid, Zinntetrachlorid, Titantetrachlorid und Antimonpentafluorid; oxidische saure Festkörper wie Zeolithe, Silikate, Aluminate, Alumosilikate, Tone und saure lonentauscher.

Bevorzugt ist der in Schritt a) eingesetzte saure Katalysator ausgewählt unter Salzsäure, Schwefelsäure, Methansulfonsäure, p-Toluolsulfonsäure und stark sauren Kationenaustauschern.

In einer ersten Variante erfolgt die Umsetzung in Schritt a) in Gegenwart einer Brönstedt-Säure, die vorzugsweise ausgewählt ist unter Salzsäure, Schwefelsäure, Methansulfonsäure, p-Toluolsulfonsäure. In dieser ersten Variante kann in Schritt a) ein Lösungsmittel eingesetzt werden, das vorzugsweise ausgewählt ist unter Kohlenwasserstoffen und Kohlenwasserstoffgemischen. Geeignete Lösungsmittel sind beispielsweise Hexan, Heptan, Ligroin, Petrolether, Cyclohexan, Dekalin, Toluol, Xylol und Mischungen davon. Bevorzugt wird kein Lösungsmittel eingesetzt. Bevorzugt wird der Katalysator in dieser ersten Variante in einer Menge von 0,05 bis 5 Mol-%, besonders bevorzugt von 0,1 bis 4 Mol-%, bezogen auf den Aldehyd (V) eingesetzt. Bevorzugt erfolgt die Umsetzung in Schritt a) nach dieser ersten Variante bei einer Temperatur im Bereich von 20 bis 120 °C, besonders bevorzugt 30 bis 80 °C.

In einer zweiten Variante erfolgt die Umsetzung in Schritt a) in Gegenwart eines stark sauren Kationenaustauschers. Unter dem Begriff stark saurer Kationenaustauscher wird dabei ein Kationenaustauscher in der H⁺-Form verstanden, der stark saure Gruppen aufweist. Bei den stark sauren Gruppen handelt es sich in der Regel um Sulfonsäuregruppen. Die sauren Gruppen sind in der Regel angebunden an eine Polymermatrix, die z. B. gelförmig bzw. makroporös sein kann. Eine bevorzugte Ausführungsform des erfindungsgemäßen Verfahrens ist dementsprechend dadurch gekennzeichnet, dass man einen stark sauren, Sulfonsäuregruppen aufweisenden Kationenaustauscher einsetzt. Geeignete stark saure Kationenaustauscher sind in der WO 2010/133473 und WO 2011/154330 beschrieben, worauf hier in vollem Umfang Bezug genommen wird.

Geeignet für den Einsatz in Schritt a) sind stark saure Ionenaustauscher (wie z. B. Amberlyst, Amberlite, Dowex, Lewatit, Purolite, Serdolit), die auf Polystyrol basieren und die Copolymere aus Styrol und Divinylbenzol als Trägermatrix mit Sulfonsäuregruppen in H⁺-Form enthalten sowie mit Sulfonsäuregruppen (-SO₃H) funktionalisierte Ionenaustauschergruppen. Die Ionenaustauscher unterscheiden sich im Aufbau ihrer Polymergerüste, und man unterscheidet gelförmige und makroporöse Harze. In einer speziellen Ausführung wird in Schritt a) ein perfluoriertes polymeres lonenaustauscherharz eingesetzt. Derartige Harze werden z. B. unter der Bezeichnung Nafion ® von der Firma DuPont vertrieben. Als Beispiel für ein solches perfluoriertes polymeres Ionenaustauscherharz sein Nafion ® NR-50 genannt.

Für die Umsetzung in Schritt a) geeignete kommerziell verfügbare stark saure Kationenaustauscher sind beispielsweise unter den Handelsnamen Lewatit ® (Lanxess), Purolite ® (The Purolite Company), Dowex ® (Dow Chemical Company), Amberlite ® (Rohm and Haas Company), Amberlyst (TM) (Rohm and Haas Company) bekannt. Bevorzugte stark saure Kationenaustauscher sind: Lewatit ® K 1221, Lewatit ® K 1461, Lewatit ® K 2431, Lewatit ® K 2620, Lewatit ® K 2621, Lewatit ® K 2629, Lewatit ® K 2649, Amberlite ® FPC 22, Amberlite ® FPC 23, Amberlite ® IR 120, Amberlyst (TM) 131, Amberlyst (TM) 15, Amberlyst (TM) 31, Amberlyst (TM) 35, Amberlyst (TM) 36, Amberlyst (TM) 39, Amberlyst (TM) 46, Amberlyst (TM) 70, Purolite ® SGC650, Purolite ® C100H, Purolite ® C150H, Dowex ® 50X8, Serdolit ® rot und Nation ® NR-50.

Die stark sauren lonentauscherharze werden in der Regel mit Salzsäure und/oder Schwefelsäure regeneriert.

In einer speziellen Ausführung werden in Schritt a) das 3-Methylbut-3-en-ol (IV) und der Aldehyd (V) in Gegenwart eines stark sauren Kationenaustauschers und in Gegenwart von Wasser umgesetzt. Nach einer speziellen Ausführung wird dem Reaktionsgemisch neben Isoprenol (IV) und dem Aldehyd der Formel (V) zusätzlich noch Wasser zugesetzt.

In einer geeigneten Ausgestaltung werden die Ausgangsstoffe in Gegenwart von mindestens 25 Mol-%, bevorzugt von mindestens 50 Mol-% Wasser umgesetzt. Beispielsweise werden die Ausgangsstoffe in Gegenwart von 25 bis 150 Mol-%, bevorzugt von 40 bis 150 Mol-%, besonders bevorzugt von 50 bis 140 Mol-%, insbesondere von 50 bis 80 Mol-% Wasser umgesetzt. Dabei bezieht sich die Menge an eingesetztem Wasser auf die Stoffmenge des gegebenenfalls im Unterschuss eingesetzten Ausgangsstoffes oder im Fall einer äquimolaren Umsetzung auf die Stoffmenge eines der beiden.

Vorzugsweise führt man die Umsetzung in Gegenwart von etwa mindestens 3 Gew.-%, besonders bevorzugt von mindestens 5 Gew.-% zugesetztem Wasser durch. Der Alkohol der Formel (IV) und der Aldehyd der Formel (V) werden beispielsweise in Gegenwart von 3 Gew.-% bis 15 Gew.-% Wasser, bevorzugt von 5 Gew.-% bis 12 Gew.-% Wasser umgesetzt. Die angegebenen vorstehenden Gew.-% sind dabei bezogen auf die Gesamtmenge des Reaktionsgemisches, bestehend aus den Komponenten der Formeln (IV) und (V) sowie Wasser.

Oberhalb des angegebenen Wertes kann die Menge an Wasser frei gewählt werden und ist, wenn überhaupt, nur durch verfahrenstechnische oder ökonomische Aspekte begrenzt und kann durchaus in großem, beispielsweise in 5- bis 15-fachem Überschuss oder auch darüber, eingesetzt werden. Vorzugsweise bereitet man ein Gemisch aus Isoprenol (IV) und dem Aldehyd der Formel (V), vorzugsweise Isovaleraldehyd, mit der zuzusetzenden Menge Wasser, so dass das zugegebene Wasser in dem Gemisch aus Isoprenol und dem Aldehyd gelöst bleibt, d. h. kein zweiphasiges System vorliegt.

Zur Umsetzung von Isoprenol (IV) mit dem Aldehyd (V) in Schritt a) kann man die genannten Ausgangsstoffe und gegebenenfalls das zugesetzte Wasser mit dem sauren Kationenaustauscher in Kontakt bringen. Vorzugsweise werden Isoprenol (IV), Aldehyd (V) und gegebenenfalls das zugesetzte Wasser in Form eines Gemisches in Schritt a) eingesetzt. Die genannten Ausgangsstoffe, d. h. Isoprenol (IV) und der Aldehyd (V) und das in der vorstehenden Menge einzusetzende Wasser, können in beliebiger Reihenfolge miteinander in Kontakt gebracht bzw. gemischt werden.

Die Menge an stark saurem Kationenaustauscher in Schritt a) ist nicht kritisch und kann unter Berücksichtigung des wirtschaftlichen und verfahrenstechnischen Aspektes in breiten Grenzen frei gewählt werden. Die Umsetzung kann dementsprechend sowohl in Gegenwart katalytischer Mengen als auch in Gegenwart großer Überschüsse des stark sauren Kationenaustauschers durchgeführt werden. Üblicherweise setzt man den stark sauren Kationentauscher in einer Menge von etwa 5 bis etwa 40 Gew.-%, bevorzugt in einer Menge von etwa 20 bis etwa 40 Gew.- % und besonders bevorzugt in einer Menge von etwa 20 bis etwa 30 Gew.-%, jeweils bezogen auf die Summe an eingesetztem Isoprenol (IV) und Aldehyd der Formel (IV), ein. Dabei beziehen sich die Angaben auf den gebrauchsfertigen Kationenaustauscher, der in der Regel mit Wasser vorbehandelt wird und dementsprechend Mengen von bis zu etwa 70 Gew.-%, bevorzugt von etwa 30 bis etwa 65 Gew.-% und besonders bevorzugt von etwa 40 bis etwa 65 Gew.-% Wasser umfassen kann. Insbesondere bei diskontinuierlicher Verfahrensführung kann sich daher ein darüber hinausgehender Wasserzusatz bei der Durchführung des erfindungsgemäßen Verfahrens erübrigen. Die genannten stark sauren Kationenaustauscher können in Schritt a) sowohl einzeln oder auch in Form von Gemischen eingesetzt werden.

Bei kontinuierlicher Fahrweise liegt die Katalysatorbelastung beispielsweise im Bereich von 50 bis 2500 mol pro m³ Katalysator und h, bevorzugt im Bereich von 100 bis 2000 mol pro m³ Katalysator und h, insbesondere im Bereich von 130 bis 1700 mol pro m³ Katalysator und h, wobei sich die Stoffmenge in mol auf den Ausgangsstoff der Formel (IV) bezieht.

Die Umsetzung in Schritt a) in Gegenwart eines stark sauren Kationenaustauschers kann wahlweise auch zusätzlich in Gegenwart eines unter den Reaktionsbedingungen inerten Lösungsmittels durchgeführt werden. Geeignete Lösungsmittel sind beispielsweise tert-Butylmethylether, Cyclohexan, Dekalin, Hexan, Heptan, Ligroin, Petrolether, Toluol oder Xylol. Die genannten Lösungsmittel können alleine oder in Form von Gemischen untereinander eingesetzt werden. Bevorzugt führt man die Umsetzung in Schritt a) in Gegenwart eines stark sauren Kationenaustauschers ohne Zusatz eines organischen Lösungsmittels durch.

Bevorzugt wird die Umsetzung von Isoprenol (IV) mit dem gewählten Aldehyd (V) in Schritt a) in Gegenwart von Wasser und in Gegenwart eines stark sauren Kationentauschers bei einer Temperatur im Bereich von 0 bis 70 °C, besonders bevorzugt bei einer Temperatur im Bereich von 20 bis 70 °C und insbesondere bei einer Temperatur im Bereich von 20 bis 60 °C durchgeführt. Dabei handelt es sich um die Temperatur des Reaktionsgemisches.

Die Umsetzung in Schritt a) kann diskontinuierlich oder kontinuierlich durchgeführt werden. Dabei kann man beispielsweise im diskontinuierlichen Fall die Umsetzung so vornehmen, dass man ein Gemisch von Isoprenol (IV), dem Aldehyd (V), gegebenenfalls Wasser und gegebenenfalls einem organischen Lösungsmittel in einem geeigneten Reaktionsgefäß vorlegt und den sauren Katalysator zugibt. Nach Abschluss der Reaktion kann dann der Katalysator durch geeignete Trennverfahren vom erhaltenen Reaktionsgemisch abgetrennt werden. Die Reihenfolge des Inkontaktbringens der einzelnen Reaktionskomponenten ist nicht kritisch und kann nach Maßgabe der jeweiligen verfahrenstechnischen Ausgestaltung variiert werden. Wird in Schritt a) eine Brönstedt-Säure, die vorzugsweise ausgewählt ist unter Salzsäure, Schwefelsäure, Methansulfonsäure, p-Toluolsulfonsäure, als Katalysator eingesetzt, so kann die Abtrennung des Katalysators z. B. nach wässriger Aufarbeitung destillativ erfolgen oder direkt destillativ erfolgen. Wird in Schritt a) ein stark saurer Kationenaustauscher als Katalysator eingesetzt, so kann die Abtrennung des Katalysators z. B. durch Filtration oder durch Zentrifugation erfolgen.

Im Rahmen einer bevorzugten Ausführungsform führt man die Umsetzung von Isoprenol (IV) mit dem Aldehyd (V) in Schritt a) kontinuierlich durch. Dazu kann beispielsweise eine Mischung der umzusetzenden Ausgangsstoffe Isoprenol und Aldehyd der Formel (V) mit Wasser bereitet werden und diese Mischung kontinuierlich mit einem stark sauren Kationentauscher in Kontakt gebracht werden. Dazu kann der gewählte Kationenaustauscher beispielsweise in einen geeigneten Durchflussreaktor, beispielsweise einen Rührreaktor mit Zu- und Ablauf oder einen Rohrreaktor eingebracht werden und die Ausgangsstoffe und das Wasser in diesen kontinuierlich eingetragen werden und das Reaktionsgemisch kontinuierlich ausgetragen werden. Dabei können die Ausgangsstoffe und das Wasser wahlweise als Einzelkomponenten oder auch in Form eines wie vorstehend beschriebenen Gemisches in den Durchflussreaktor eingetragen werden. Entsprechende Verfahren sind in den europäischen Patentanmeldungen 13165767.8 und 13165778.5 beschrieben.

In Schritt a) des erfindungsgemäßen Verfahrens wird ein Reaktionsgemisch erhalten, das
- wenigstens ein 2-substituiertes 4-Hydroxy-4-methyl-tetrahydropyran der allgemeinen Formel (I),
- wenigstens eine Dioxanverbindung (II) 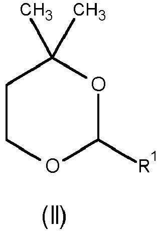
- und wenigstens eine der Verbindungen (III.1), (III.2) oder (III.3) 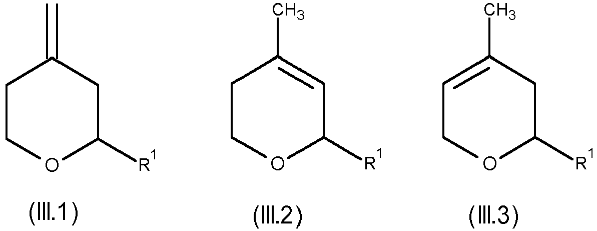
enthält, worin
- R¹: für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht.

Bevorzugt steht R¹ für Isobutyl. In der Regel enthält das Reaktionsgemisch ein Gemisch der Verbindungen (III.1), (III.2) und (III.3).

Das in Schritt a) des erfindungsgemäßen Verfahrens erhaltene Reaktionsgemisch kann wenigstens eine Verbindung enthalten, die vorzugsweise ausgewählt ist unter:
- 3-Methylbut-3-en-1-olen der Formel (IV)
- Aldehyden der Formel (V)
- Acetalen der allgemeinen Formel (VI) 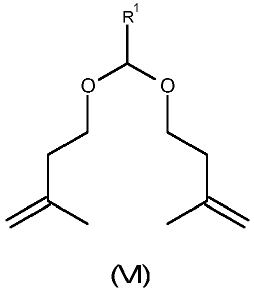
worin
- R¹: für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht.

Bevorzugt steht R¹ für Isobutyl.

Das in Schritt a) des erfindungsgemäßen Verfahrens erhaltene Reaktionsgemisch kann weitere Komponenten enthalten, wie Wasser, organisches Lösungsmittel, etc.

Vorzugsweise enthält das in Schritt a) erhaltene Reaktionsgemisch das 2-substituierte 4-Hydroxy-4-methyl-tetrahydropyran der Formel (I) in einer Menge von 50 bis 90 Gew.-%, besonders bevorzugt 60 bis zu etwa 80 Gew.-%, bezogen auf das Gesamtgewicht des Reaktionsgemischs.

Vorzugsweise enthält das in Schritt a) erhaltene Reaktionsgemisch die DioxanVerbindung der Formel (II) in einer Gesamtmenge von 5 bis 20 Gew.-%, besonders bevorzugt 5 bis zu etwa 15 Gew.-%, bezogen auf das Gesamtgewicht des Reaktionsgemischs.

Vorzugsweise enthält das in Schritt a) erhaltene Reaktionsgemisch die Verbindungen der Formeln (III.1), (III.2) und (III.3) in einer Gesamtmenge von 5 bis 20 Gew.-%, besonders bevorzugt 5 bis zu etwa 15 Gew.-%, bezogen auf das Gesamtgewicht des Reaktionsgemischs.

In einer typischen Zusammensetzung enthält das in Schritt a) erhaltene Reaktionsgemisch die folgenden Verbindungen, jeweils bezogen auf das Gesamtgewicht des Reaktionsgemischs:
60 bis 85 Gew.-% wenigstens einer Verbindung (I),
5 bis 15 Gew.-% wenigstens einer Verbindung (II),
5 bis 15 Gew.-% wenigstens einer der Verbindungen (III.1), (III.2) oder (III.3),
0 bis 10 Gew.-% wenigstens eines 3-Methylbut-3-en-1-ols (IV),
0 bis 5 Gew.-% wenigstens eines Aldehyds (V),
0 bis 5 Gew.-% wenigstens einer Verbindung (VI),
2 bis 10 Gew.-% Wasser.

Bevorzugt enthält das in Schritt a) erhaltene Reaktionsgemisch:
15 bis 22 Gew.-% trans-(I),
45 bis 65 Gew.-% cis-(I),
5 bis 15 Gew.-% wenigstens einer Verbindung (II),
5 bis 15 Gew.-% wenigstens einer der Verbindungen (III.1), (III.2) oder (III.3),
0 bis 10 Gew.-% wenigstens eines 3-Methylbut-3-en-1-ols (IV),
0 bis 5 Gew.-% wenigstens eines Aldehyds (V),
0 bis 5 Gew.-% wenigstens einer Verbindung (VI),
2 bis 10 Gew.-% Wasser,
jeweils bezogen auf das Gesamtgewicht des Reaktionsgemischs.

Bevorzugt enthält das in Schritt a) erhaltene Reaktionsgemisch die 2-substituierten 4-Hydroxy-4-methyl-tetrahydropyrane der Formel (I) in Form von Gemischen der cis-Diastereomeren der Formel cis-(I) und der trans-Diastereomeren der Formel trans-(I) 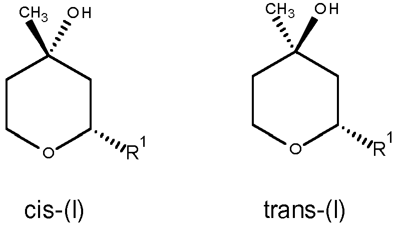 wobei das Diastereomerenverhältnis des cis-Diastereomeren cis-(I) zum trans-Diastereomeren trans-(I) bevorzugt 65 zu 35 bis 95 zu 5, besonders bevorzugt 70 zu 30 bis 85 zu 15 beträgt und R¹ die weiter oben angegebenen Bedeutungen hat.

Bevorzugt enthält das in Schritt a) erhaltene Reaktionsgemisch 2-Isobutyl-4-hydroxy-4-methyl-tetrahydropyran in Form von Gemischen des cis-Diastereomeren der Formel cis-(I.a) und des trans-Diastereomeren der Formel trans-(I.a) 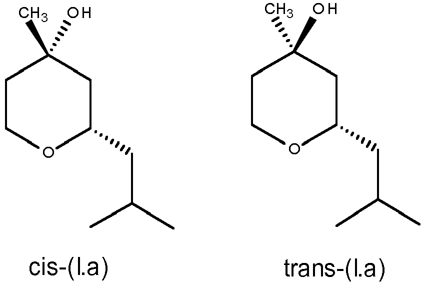 wobei das Diastereomerenverhältnis des cis-Diastereomeren cis-(I.a) zum trans-Diastereomeren trans-(I.a) bevorzugt 65 zu 35 bis 95 zu 5, besonders bevorzugt 70 zu 30 bis 85 zu 15 beträgt.

Derartige Gemische eignen sich aufgrund ihrer besonderen geruchlichen Eigenschaften in besonderem Masse zur Verwendung als Aromachemikalien, beispielsweise als Komponente mit Maiglöckchenduft zur Herstellung von Riechstoffkompositionen.

### Säurebehandlung

Erfindungsgemäß wird das Reaktionsgemisch aus Schritt a) vor dem Einsatz in Schritt b) und/oder während des Einsatzes in Schritt b) mit einem sauren Ionenaustauscher in Kontakt gebracht und/oder mit einer starken Säure versetzt.

In einer ersten Variante erfolgt die Behandlung des Reaktionsgemisches aus Schritt a) mit wenigstens einer sauren Komponente in homogener Phase. Bei der erfindungsgemäßen Behandlung in homogener Phase liegen die wechselwirkenden Komponenten in der flüssigen Phase vor. Dazu kann ein flüssiges Reaktionsgemisch aus der Umsetzung von 3-Methylbut-3-en-1-ol (IV) mit einem Aldehyd (V) oder eine durch destillative Auftrennung eines solchen Reaktionsgemisches erhaltene flüssige Fraktion mit einer starken Säure versetzt werden, die unter den Behandlungsbedingungen mit dem Reaktionsgemisch oder einer Fraktion des Reaktionsgemisches zumindest teilweise mischbar oder in diesem zumindest teilweise löslich ist.

In einer zweiten Variante erfolgt die Behandlung des Reaktionsgemisches aus Schritt a) mit wenigstens einer sauren Komponente in heterogener Phase. Bei der erfindungsgemäßen Behandlung in heterogener Phase liegen die wechselwirkenden Komponenten in der Regel teilweise in der flüssigen Phase und teilweise in der festen Phase vor. Dazu kann ein flüssiges Reaktionsgemisch aus der Umsetzung von 3-Methylbut-3-en-1-ol (IV) mit einem Aldehyd (V) oder eine durch destillative Auftrennung eines solchen Reaktionsgemisches erhaltene flüssige Fraktion mit einer in fester Form vorliegenden Säure in Kontakt gebracht werden. Bevorzugt wird die Säure zur Behandlung in fester Phase in Form eines Festbetts eingesetzt. Bevorzugt wird zur Behandlung in fester Phase ein saurer Ionenaustauscher eingesetzt.

Geeignete starke Säuren und saure Ionenaustauscher sind die zuvor in Schritt a) genannten sauren Katalysatoren. Darauf wird hier in vollem Umfang Bezug genommen.

Bevorzugt wird nach der ersten Variante das Reaktionsgemisch aus Schritt a) vor dem Einsatz in Schritt b) oder eine bei der destillativen Auftrennung in Schritt b) erhaltene Fraktion mit einer starken Säure versetzt. In einer bevorzugten Ausführung wird das Reaktionsgemisch aus Schritt a) vor dem Einsatz in Schritt b) mit einer starken Säure versetzt. Alternativ ist es möglich, den Sumpf der destillativen Auftrennung in Schritt b) oder bei einer mehrstufigen Destillation den Sumpf der ersten Destillationsstufe der destillativen Auftrennung in Schritt b) mit einer starken Säure zu versetzen.

Bevorzugt wird das Reaktionsgemisch aus Schritt a) nach der ersten Variante mit einer starken Säure versetzt, die ausgewählt ist unter Schwefelsäure, Salzsäure, Methansulfonsäure und p-Toluolsulfonsäure.

Besonders bevorzugt wird das Reaktionsgemisch aus Schritt a) vor dem Einsatz in Schritt b) mit Schwefelsäure versetzt.

Vorzugsweise versetzt man das Reaktionsgemisch aus Schritt a) vor dem Einsatz in Schritt b) mit 1 bis 250 Gew.-ppm, bevorzugt mit 2 bis 100 Gew.-ppm, bezogen auf das Gesamtgewicht des Reaktionsgemischs, einer starken Säure. Alternativ kann man den Sumpf der destillativen Auftrennung in Schritt b) oder bei einer mehrstufigen Destillation den Sumpf der ersten Destillationsstufe der destillativen Auftrennung in Schritt b) mit 1 bis 250 Gew.-ppm, bevorzugt mit 2 bis 100 Gew.-ppm, bezogen auf das Gesamtgewicht des Sumpfprodukts, einer starken Säure versetzen. Die Mengenangabe bezieht sich dabei auf die reine Säure. Selbstverständlich ist es möglich und vielfach bevorzugt, die Säure in verdünnter Form, speziell als wässrige Lösung, einzusetzen.

Nach der zuvor beschriebenen zweiten Variante bringt man das Reaktionsgemisch aus Schritt a) vor dem Einsatz in Schritt b) mit einem sauren Ionenaustauscher in Kontakt.

Das Inkontaktbringen mit dem sauren Ionenaustauscher kann diskontinuierlich oder kontinuierlich erfolgen. Bevorzugt ist das kontinuierliche Inkontaktbringen.

Bevorzugt erfolgt das Inkontaktbringen bei einer Temperatur von 30 bis 80 °C, besonders bevorzugt von 40 bis 70 °C.

Geeignete Ionenaustauscher sind die zuvor genannten stark sauren Kationenaustauscher. Die Einsatzmenge an saurem Ionenaustauscher ist dabei in der Regel nicht kritisch. Die Umsetzung kann dementsprechend sowohl in Gegenwart katalytischer Mengen als auch in Gegenwart eines Überschusses eines stark sauren Kationenaustauschers durchgeführt werden.

Insbesondere wird das Reaktionsgemisch aus Schritt a) vor dem in Kontakt bringen mit einem sauren Ionenaustauscher und/oder dem Versetzen mit einer starken Säure keiner destillativen Auftrennung unterzogen.

### Schritt b)

Bevorzugt wird in Schritt b) des erfindungsgemäßen Verfahrens das Reaktionsgemisch aus Schritt a) einer destillativen Auftrennung unterzogen. Geeignete Vorrichtungen zur destillativen Auftrennung umfassen Destillationskolonnen, wie Bodenkolonnen, die mit Glocken, Siebplatten, Siebböden, Packungen, Füllkörpern, Ventilen, Seitenabzügen, etc. ausgerüstet sein können, Verdampfer, wie Dünnschichtverdampfer, Fallfilmverdampfer, Zwangsumlaufverdampfer, Sambay-Verdampfer, etc., und Kombinationen davon.

Die Destillationskolonnen können trennwirksame Einbauten aufweisen, die vorzugsweise ausgewählt sind unter Trennböden, geordnete Packungen, z. B. Blech- oder Gewebepackungen, wie Sulzer Mellapak®, Sulzer BX, Montz B1 oder Montz A3 oder Kühni Rombopak, oder regellose Schüttungen von Füllkörpern, wie z. B. Dixon-Ringen, Raschig-Ringen, High-Flow-Ringen oder Raschig-Super-Ringen. Besonders bewährt haben sich geordnete Packungen, vorzugsweise Blech- oder Gewebepackungen, mit einer spezifischen Oberfläche von 100 bis 750 m²/m³, insbesondere 250 bis 500 m²/m³. Sie gestalten hohe Trennleistungen bei niedrigen Druckverlusten.

Vorzugsweise wird zur Auftrennung in Schritt b) eine Vorrichtung eingesetzt, die
- eine Zulaufsäule mit oberhalb der Zulaufstelle gelegenem Verstärkungsteil und unterhalb der Zulaufstelle gelegenem Abtriebsteil,
- eine mit dem oberen Ende des Verstärkungsteils kommunizierende obere Vereinigungssäule und eine mit dem unteren Ende des Abtriebsteils kommunizierende untere Vereinigungssäule, und
- eine mit der oberen Vereinigungssäule und der unteren Vereinigungssäule kommunizierende Abzugssäule
umfasst.

Vorzugsweise erfolgt die Auftrennung in Schritt b), indem man
i) das Reaktionsprodukt aus Schritt a) in eine Zulaufsäule mit oberhalb der Zulaufstelle gelegenem Verstärkungsteil und unterhalb der Zulaufstelle gelegenem Abtriebsteil einführt,
ii) eine mit dem oberen Ende des Verstärkungsteils kommunizierende obere Vereinigungssäule mit Kondensator am oberen Säulenende und eine mit dem unteren Ende des Abtriebsteils kommunizierende untere Vereinigungssäule mit Aufheizer am unteren Säulenende vorsieht,
iii) eine mit der oberen Vereinigungssäule und der unteren Vereinigungssäule kommunizierende Abzugssäule vorsieht, die wenigstens einen Seitenabzug aufweist,
iv) aus der Abzugssäule am Kopf oder im oberen Bereich leichter als die 2-substituierten 4-Hydroxy-4-methyl-tetrahydropyrane (I) siedende Verbindungen abzieht, als wenigstens ein Seitenabzug zumindest einen Teil der 2-substituierten 4-Hydroxy-4-methyl-tetrahydropyrane (I) abzieht und im Sumpf oder im unteren Bereich der unteren Vereinigungssäule die 2-substituierten 4-Hydroxy-4-methyl-tetrahydropyrane (I) abzieht, die nicht als Seitenabzug abgezogen werden und die höher als die 2-substituierten 4-Hydroxy-4-methyl-tetrahydropyrane (I) siedenden Verbindungen abzieht.

In einer bevorzugten Ausführungsform enthält der aus der Abzugssäule am Kopf oder im oberen Bereich entnommene Abzug:
- zumindest einen Teil oder die Gesamtmenge der in dem Reaktionsprodukt aus Schritt a) enthaltenen Dioxanverbindung (II),
- zumindest einen Teil oder die Gesamtmenge der in dem Reaktionsprodukt aus Schritt a) enthaltenen Verbindungen (III.1), (III.2) und (III.3),
- falls vorhanden, nicht umgesetztes 3-Methylbut-3-en-1-ol der Formel (IV),
- falls vorhanden, nicht umgesetzten Aldehyd (V),
- geringe Mengen oder keine 2-substituierten 4-Hydroxy-4-methyl-tetrahydropyrane (I),
- Wasser.

In einer besonders bevorzugten Ausführungsform werden zur Umsetzung in Schritt a) 3-Methylbut-3-en-1-ol der Formel (IV) und Isovaleraldehyd (V) eingesetzt. Dann enthält der aus der Abzugssäule am Kopf oder im oberen Bereich entnommene Abzug:
- zumindest einen Teil oder die Gesamtmenge der in dem Reaktionsprodukt aus Schritt a) enthaltenen Dioxanverbindung (II), worin R¹ für Isobutyl steht,
- zumindest einen Teil oder die Gesamtmenge der in dem Reaktionsprodukt aus Schritt a) enthaltenen Verbindungen (III.1), (III.2) und (III.3), worin R¹ für Isobutyl steht,
- falls vorhanden, nicht umgesetztes 3-Methylbut-3-en-1-ol der Formel (IV),
- falls vorhanden, nicht umgesetzten Isovaleraldehyd (V),
- geringe Mengen oder kein 2-(2-Methylpropyl)-4-hydroxy-4-methyl-tetrahydropyran der Formel (I.a),
- Wasser.

Das so erhaltene Kopfprodukt kann einer Phasentrennung zur Abtrennung der Hauptmenge des Wassers unterzogen werden. Abgesehen von einer solchen Phasentrennung kann das so erhaltene Kopfprodukt in der Regel ohne weitere Aufarbeitung einer Weiterverarbeitung unterzogen werden. Dazu zählt eine Hydrierung zu 2-substituierten 4-Methyl-tetrahydropyranen (VII) und speziell 2-(2-Methylpropyl)-4-methyl-tetrahydropyran (Dihydrorosenoxid). Gewünschtenfalls kann das Kopfprodukt einer weiteren Aufarbeitung zur Abtrennung wenigstens eines Teils der von den Verbindungen (II), (III.1), (III.2) und (III.3) verschiedenen Komponenten unterzogen werden. Dazu kann das Kopfprodukt z. B. einer weiteren destillativen Auftrennung unterzogen werden.

In einer bevorzugten Ausführungsform wird aus der Abzugssäule ein Seitenstrom oder werden aus der Abzugssäule zwei Seitenströme abgezogen. In einer speziellen Ausführung wird aus der Abzugssäule nur ein Seitenstrom abgezogen.

Werden in Schritt b) mehrere Abzüge entnommen, die 2-substituierte 4-Hydroxy-4-methyl-tetrahydropyrane (I) enthalten, z. B. zwei verschiedene Seitenabzüge oder ein Seitenabzug und ein Sumpfabzug, so unterscheiden sich diese in der Regel hinsichtlich der Zusammensetzung der Stereoisomeren. Somit gelingt die Isolierung einer gegenüber dem Reaktionsprodukt aus Schritt a) an cis-Diastereomeren angereicherten Fraktion und einer an trans-Diastereomeren angereicherten Fraktion. Bei ausreichender Trennleistung der eingesetzten Destillationsvorrichtung kann gewünschtenfalls zumindest eines der Diastereomeren in reiner Form erhalten werden.

Die Zulaufsäule, Abzugssäule, obere Vereinigungssäule und untere Vereinigungssäule können diskrete Bauelemente sein oder als Abschnitt oder Kammer einer Destillationskolonne ausgebildet sein, die mehrere Funktionen vereint. Der Ausdruck "kommunizierende Kolonnen" bedeutet, dass zwischen ihnen ein Austausch sowohl von aufsteigenden Brüden als auch von ablaufendem Kondensat erfolgt.

In einer bevorzugten Ausführungsform des erfindungsgemäßen Verfahrens erfolgt die destillative Auftrennung in Schritt b) in einer Anordnung von Destillationskolonnen, die eine Trennwandkolonne oder eine Zusammenschaltung von mindestens zwei thermisch gekoppelten konventionellen Destillationskolonnen umfasst.

Trennwandkolonnen sind spezielle Destillationskolonnen mit mindestens einer Zulaufstelle und mindestens drei Entnahmestellen, bei denen sich zwischen Verdampfer und Kondensator der sogenannte Rektifizierbereich befindet, in dem ein Teil des im Kondensator gebildeten Kondensats sich flüssig als Rücklauf im Gegenstrom zu den aus der Verdampfungseinrichtung aufsteigenden Dämpfen abwärts bewegt und welche in einem Teilbereich der Kolonne unterhalb und/oder oberhalb der Zulaufstelle mindestens eine in Längsrichtung wirkende Trenneinrichtung (Trennwand) zur Verhinderung einer Quervermischung von Flüssigkeits- und/oder Brüdenstrom (Dampfstrom) enthält und welche somit eine destillative Trennung von Stoffgemischen ermöglichen. Das Grundprinzip der Trennwandkolonnen ist seit langem bekannt und beispielsweise in der US 2,471,134, in der EP-A-0 122 367 oder in G. Kaibel, Chem. Eng. Technol. Vol. 10, 1987, Seite 92 bis 98 beschrieben.

Der allgemeine Grundaufbau einer Trennwandkolonne umfasst mindestens eine seitliche Zulaufstelle auf einer Seite der Trennwand und mindestens drei Entnahmestellen, von denen sich mindestens eine jenseits der Trennwand befindet. Da bei dieser Bauart im Bereich der Trennwand eine Quervermischung von Flüssigkeits- und/oder Brüdenstrom verhindert wird, ist es möglich, die Seitenprodukte in reiner Form zu erhalten. Hierdurch verringert sich bei der Auftrennung von Vielstoffgemischen im Allgemeinen die Zahl der insgesamt benötigten Destillationskolonnen. Zudem können beim Einsatz von Trennwandkolonnen gegenüber einer einfachen Hintereinanderschaltung zweier konventioneller Destillationskolonnen Investitionskosten sowie Energie eingespart werden (siehe M. Knott, Process Engineering, Vol. 2, 1993, February, Seite 33 bis 34).

Als konventionelle Destillationskolonnen werden im Sinne der Erfindung alle Destillationskolonnen bezeichnet, welche keine Trennwand enthalten. Bei thermisch gekoppelten konventionellen Destillationskolonnen werden Massen- und Energieströme wechselseitig ausgetauscht. Somit ist gegenüber einer einfachen Hintereinanderschaltung konventioneller Destillationskolonnen eine deutliche Einsparung von Energie möglich. Bevorzugt als Alternative zur Trennwandkolonne ist eine Verschaltung zweier thermisch gekoppelter Destillationskolonnen. Eine Übersicht verschiedener Anordnungen ist beispielsweise in G. Kaibel et al., Chem.-Ing.-Tech., Vol. 61, 1989, Seite 16 bis 25 und G. Kaibel et al., Gas Separation & Purification, Vol. 4, 1990, June, Seiten 109 bis 114, gegeben.

In einer ersten bevorzugten Ausführungsformen verwendet man zur Destillation eine Destillationskolonne mit einer thermisch gekoppelten Vorkolonne, d. h. die Abzugssäule, die obere Vereinigungssäule und die untere Vereinigungssäule sind als einstückige Destillationskolonne ausgebildet, und die Zulaufsäule ist als Vorkolonne zur Destillationskolonne ausgebildet. In einer zweiten bevorzugten Ausführungsform verwendet man eine Destillationskolonne mit einer thermisch gekoppelten Nachkolonne, d. h. die Zulaufsäule, die obere Vereinigungssäule und die untere Vereinigungssäule sind als einstückige Destillationskolonne ausgebildet und die Abzugssäule ist als Nachkolonne zu der Destillationskolonne ausgebildet. Destillationskolonnen mit beigeschalteten Hilfskolonnen sind bekannt und z. B. in Chem. Eng. Res. Des., Part A: Trans IChemE, März 1992, S. 118-132, "The design and optimisation of fully thermally coupled distillation columns", beschrieben.

Es hat sich als günstig erwiesen, aus dem Reaktionsprodukt aus Schritt a) vor dem Einführen in die Zulaufsäule zumindest einen Teil der leichter als die 2-substituierten 4-Hydroxy-4-methyl-tetrahydropyrane (I) siedenden Verbindungen zu entfernen. In einer speziellen Ausführungsform wird daher zur destillativen Auftrennung des Reaktionsprodukts aus Schritt a) eine Anordnung von Destillationskolonnen eingesetzt, die eine vorgeschaltete konventionelle Destillationskolonne und eine nachgeschaltete Trennwandkolonne oder eine nachgeschaltete Zusammenschaltung von zwei thermisch gekoppelten konventionellen Destillationskolonnen umfasst.

Bevorzugt wird zur destillativen Auftrennung in Schritt b)
b1) das Reaktionsgemisch aus Schritt a) zunächst einer Auftrennung in einer konventionellen Destillationskolonne unterzogen, wobei ein erstes Kopfprodukt erhalten wird, das an der Dioxanverbindung (II) und den Verbindungen (III.1), (III.2) und (III.3) und angereichert ist und im Wesentlichen keine Verbindungen der allgemeinen Formel (I) enthält, und ein erstes Sumpfprodukt erhalten wird, das an der Dioxanverbindung (II) und den Verbindungen (III.1), (III.2) und (III.3) abgereichert ist und die Hauptmenge der Verbindungen der allgemeinen Formel (I) enthält,
b2) das erste Sumpfprodukt aus Schritt b1) einer Auftrennung in einer Trennwandkolonne oder in einer Zusammenschaltung von zwei thermisch gekoppelten konventionellen Destillationskolonnen unterzogen, wobei ein zweites Kopfprodukt erhalten wird, das die nicht im ersten Kopfprodukt enthaltenen Verbindungen (II), (III.1), (III.2), (III.3) und sowie gegebenenfalls geringe Mengen der Verbindungen der allgemeinen Formel (I) enthält, ein Seitenstrom erhalten wird, der im Wesentlichen aus Verbindung der allgemeinen Formel (I) besteht und ein zweites Sumpfprodukt erhalten wird, das die Verbindungen der allgemeinen Formel (I) enthält, die nicht im Kopfprodukt und nicht im Seitenstrom enthalten sind.

Bevorzugt steht auch in der zuvor genannten Ausführungsform in den Verbindungen der Formeln (I), (II), (III.1), (III.2) und (III.3) R¹ für Isobutyl.

Der Ausdruck, wonach das erste Kopfprodukt im Wesentlichen keine Verbindungen der allgemeinen Formel (I) enthält, bedeutet, dass der Anteil Verbindungen der allgemeinen Formel (I) am ersten Kopfprodukt höchstens 5 Gew.-%, besonders bevorzugt höchstens 2 Gew.-%, insbesondere höchstens 1 Gew.-%, speziell höchstens 0,1 Gew.-%, bezogen auf das Gesamtgewicht des ersten Kopfprodukts, beträgt. In einer speziellen Ausführung enthält das erste Kopfprodukt keine Verbindungen der allgemeinen Formel (I).

Das zweite Kopfprodukt kann beispielsweise 1 bis40 Gew.-%, besonders bevorzugt 2 bis 30 Gew.-%, insbesondere 5 bis 25 Gew.-%, speziell 10 bis 20 Gew.-%, Verbindungen der allgemeinen Formel (I), bezogen auf das Gesamtgewicht des zweiten Kopfprodukts, enthalten.

In einer speziellen Ausführung besteht der Seitenstrom nur aus Verbindungen der allgemeinen Formel (I).

In einer weiteren speziellen Ausführung kann das zweite Sumpfprodukt Verbindungen enthalten, die höher sieden als die Verbindungen der allgemeinen Formel (I).

Die in Schritt b) erhaltene(n) Fraktion(en) der 2-substituierten 4-Hydroxy-4-methyl-tetrahydropyrane der allgemeinen Formel (I) zeichnen sich vorteilhafterweise durch einen gegenüber dem Ausgangsmaterial deutlich verringerten Gehalt an Komponenten aus, die den Geruchseindruck negativ beeinflussen. Insbesondere kann das Auftreten von käsigen Noten wirkungsvoll verhindert werden. Die in Schritt b) erhaltene(n) Fraktionen) der 2-substituierten 4-Hydroxy-4-methyl-tetrahydropyrane der allgemeinen Formel (I) können in vielen Fällen bereits ohne weitere Aufarbeitung einer kommerziellen Verwendung zugeführt werden.

Gewünschtenfalls können die in Schritt b) erhaltene(n) Fraktion(en) der 2-substituierten 4-Hydroxy-4-methyl-tetrahydropyrane der allgemeinen Formel (I) einer weiteren Aufarbeitung unterzogen werden. Dazu können die Fraktionen einzeln oder nach einer (teilweisen) Vereinigung eingesetzt werden. Geeignet sind die üblichen, dem Fachmann bekannten Reinigungsverfahren. Dazu zählt beispielsweise eine Destillation, eine Extraktion oder eine Kombination davon.

Eine spezielle Ausführungsform betrifft ein Verfahren, bei dem man das mit Säure behandelte Reaktionsgemisch aus Schritt a) einer destillativen Auftrennung in einer Zusammenschaltung von Destillationskolonnen unterzieht, die
- eine konventionelle Destillationskolonne,
- eine erste Trennwandkolonne oder eine erste Zusammenschaltung von zwei thermisch gekoppelten konventionellen Destillationskolonnen, und
- eine zweite Trennwandkolonne oder eine zweite Zusammenschaltung von zwei thermisch gekoppelten konventionellen Destillationskolonnen,
umfasst.

Eine weitere spezielle Ausführungsform betrifft ein Verfahren, bei dem man das mit Säure behandelte Reaktionsgemisch aus Schritt a)
- einer ersten destillativen Auftrennung unterzieht, wobei ein erstes Kopfprodukt erhalten wird, das an der Dioxanverbindung (II) und den Verbindungen (III.1), (III.2) und (III.3) und angereichert ist und im Wesentlichen keine Verbindungen der allgemeinen Formel (I) enthält, und ein erstes Sumpfprodukt erhalten wird, das an der Dioxanverbindung (II) und den Verbindungen (III.1), (III.2) und (III.3) abgereichert ist und die Hauptmenge der Verbindungen der allgemeinen Formel (I) enthält,
- das erste Sumpfprodukt mit wenigstens einer Base versetzt oder einer einfachen Destillation unterzieht, wobei die Hauptmenge des ersten Sumpfprodukts verdampft und anschließend kondensiert wird,
- das mit Base versetzte erste Sumpfprodukt oder das Kondensat einer weiteren destillativen Auftrennung unterzogen wird.

Geeignete Basen für die Zugabe zum ersten Sumpfprodukt sind Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxid, Calciumoxid, Magnesiumhydroxid oder Magnesiumcarbonat sowie Amine. Besonders bevorzugt ist die Base ausgewählt unter Na₂CO₃, K₂CO₃, NaHCO₃, KHCO₃, NaOH, KOH und Mischungen davon.

Bevorzugt weist das erste Sumpfprodukt nach der Basenzugabe einen pH-Wert im Bereich von 4 bis 7 auf.

In einer speziellen Ausführungsform wird das erste Sumpfprodukt mit einem Bett wenigstens einer Base in Kontakt gebracht.

Unter einer einfachen Destillation wird im Rahmen der Erfindung eine Destillation verstanden, bei der nicht wie bei der Rektifikation oder Gegenstromdestillation ein Teil des Kondensats im Gegenstrom zu den aufsteigenden Dämpfen der siedenden Mischung wieder zurückgeführt wird, sondern wobei das Sumpfprodukt größtenteils verdampft und anschließend kondensiert wird. In einer bevorzugten Ausführungsform wird ein Sambay-Verdampfer eingesetzt.

Die Menge des ersten Sumpfprodukts, welche verdampft wird, umfasst vorzugsweise 60 bis 99,9 Gew.-%, besonders bevorzugt 75 bis 99 Gew.-%, insbesondere 90 bis 98 Gew.-% der Gesamtmenge des Sumpfprodukts.

Das mit Base versetzte erste Sumpfprodukt oder das Kondensat wird vorzugsweise einer weiteren destillativen Auftrennung in einer Trennwandkolonne oder in einer Zusammenschaltung von zwei thermisch gekoppelten konventionellen Destillationskolonnen unterzogen. Dabei wird vorzugsweise ein zweites Kopfprodukt erhalten, das die nicht im ersten Kopfprodukt enthaltenen Verbindungen (II), (III.1), (III.2), (III.3) und sowie gegebenenfalls geringe Mengen der Verbindungen der allgemeinen Formel (I) enthält, ein Seitenstrom, der im Wesentlichen aus Verbindung der allgemeinen Formel (I) besteht und ein zweites Sumpfprodukt, das die Verbindungen der allgemeinen Formel (I) enthält, die nicht im Kopfprodukt und nicht im Seitenstrom enthalten sind.

Die erfindungsgemäßen Zusammensetzungen und die nach dem erfindungsgemäßen Verfahren erhältlichen Zusammensetzungen eignen sich besonders vorteilhaft als Riechstoff oder zur Bereitstellung eines Riechstoffs.

Dabei können zusätzlich zu den 2-substituierten 4-Hydroxy-4-methyl-tetrahydropyranen der allgemeinen Formel (I) auch die in Schritt b) erhaltene Fraktion, an wenigstens einer der Verbindungen (III.1), (III.2) oder (III.3) angereichert ist, einer Weiterverarbeitung zur Bereitstellung eines Riechstoffs unterzogen werden. So wird durch Hydrierung der Verbindungen (III.1), (III.2) oder (III.3) ein Hydrierprodukt erhalten, das wenigstens ein 2-substituiertes 4-Methyl-tetrahydropyran der allgemeinen Formel (VII) 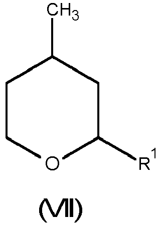 enthält, worin
- R¹: für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht.

Insbesondere steht R¹ für Isobutyl. Diese, als Dihydrorosenoxid bezeichnete Verbindung eignet sich aufgrund ihrer besonderen Geruchseigenschaften mit rosenduftartigem Charakter in besonderem Maße zur Verwendung als Aromachemikalie und speziell zur Herstellung von Riechstoffkompositionen.

Vorteilhafterweise ermöglicht diese spezielle Ausführungsform ein integriertes Verfahren zur gleichzeitigen Herstellung von 2-substituierten 4-Hydroxy-4-methyl-tetrahydropyranen (I) und von 2-substituierten 4-Methyl-tetrahydropyranen (VII).

Die vorliegende Erfindung betrifft somit im Rahmen einer speziell bevorzugten Ausführungsform ein Verfahren zur Herstellung und Isolierung von 2-(2-Methylpropyl)-4-hydroxy-4-methyl-tetrahydropyran der Formel (I.a) und von Dihydrorosenoxid (VII.a)

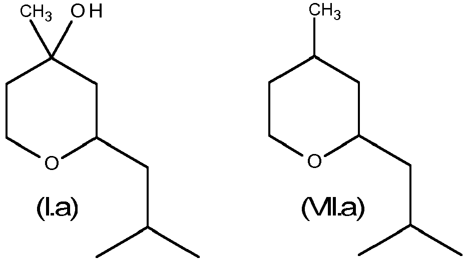

Die erfindungsgemäßen Zusammensetzungen können für den Einsatz als Riechstoff mit wenigstens einem auf diesem Anwendungsgebiet üblichen Lösemittel beliebig verdünnt werden. Beispielhaft seien als geeignete Lösemittel genannt: Ethanol, Dipropylenglycol oder dessen Ether, Phthalate, Propylenglykole, oder Carbonate von Diolen, bevorzugt Ethanol. Auch Wasser ist als Lösemittel zur Verdünnung der erfindungsgemäßen Duftstoffkompositionen geeignet und kann vorteilhaft zusammen mit geeigneten Emulgatoren eingesetzt werden.

Die nach dem erfindungsgemäßen Verfahren erhaltenen Riechstoffe weisen auf Grund der strukturellen und chemischen Ähnlichkeit der Komponenten eine hohe Stabilität und Haltbarkeit auf. Die nach dem erfindungsgemäßen Verfahren erhältlichen Isomerengemische von 2-(2-Methylpropyl)-4-hydroxy-4-methyl-tetrahydropyran der Formel (I.a) zeichnen sich durch einen angenehmen Maiglöckchengeruch aus. Die nach dem erfindungsgemäßen Verfahren erhältlichen Isomerengemische von 2-(2-Methylpropyl)-4-methyl-tetrahydropyran der Formel (VII.a) (Dihydrorosenoxid) zeichnen sich durch einen angenehmen rosenartigem Charakter aus.

Die nach dem erfindungsgemäßen Verfahren erhaltenen Riechstoffe eigenen sich zur Einarbeitung in kosmetische Zusammensetzungen sowie Gebrauchs- und Verbrauchsgüter bzw. Mittel wie sie im Folgenden näher beschrieben sind, wobei die Riechstoffe in die genannten Güter eingearbeitet oder auch auf solche aufgebracht sein können. Unter einer organoleptisch wirksamen Menge ist dabei wie im Rahmen der gesamten vorliegenden Erfindung insbesondere eine solche Menge zu verstehen, die bei sachgemäßer Anwendung ausreicht, beim Anwender bzw. Verbraucher einen Dufteindruck hervorzurufen.

Als kosmetische Zusammensetzungen sind alle üblichen kosmetischen Zusammensetzungen geeignet. Dabei handelt es sich bevorzugt um Parfum, Eau de Toilette, Deodorants, Seife, Duschgel, Badegel, Cremes, Lotions, Sonnenschutzmittel, Zusammensetzungen zur Reinigung und Pflege der Haare wie Haarshampoo, Spülung, Haargel, Haarfestiger in Form von Flüssigkeiten oder Schäumen und weitere Reinigungs- oder Pflegemittel für die Haare, Zusammensetzungen zur dekorativen Anwendung am menschlichen Körper, wie kosmetische Stifte, zum Beispiel Lippenstifte, Lippenpflegestifte, Abdeckstifte (Concealer), Wangenrouge (Blusher), Lidschattenstifte, Lippenkonturenstifte, Augenkonturenstifte, Augenbrauenstifte, Korrekturstifte, Sonnenschutzstifte, Anti-Akne-Stifte und vergleichbare Produkte sowie Nagellacke und weitere Produkte zur Nagelpflege.

Die nach dem erfindungsgemäßen Verfahren erhaltenen Riechstoffe eignen sich speziell für einen Einsatz in Parfums, z. B. als Eau de Toilette, Duschgele, Badegele und Körperdeodorants.

Sie eignen sich weiterhin zur Aromatisierung von Verbrauchs- oder Gebrauchsgütern, in die sie eingearbeitet bzw. auf die sie aufgebracht werden und ihnen dadurch einen angenehmen frischen grünen Akzent verleihen. Beispiele für Verbrauchs- oder Gebrauchsgüter sind: Raumluftdeodorants (Air Care), Reinigungsmitteln oder Pflegemitteln für Textilien (speziell Waschmittel, Weichspüler), Textilbehandlungsmittel wie beispielsweise Bügelhilfsmittel, Putzmittel, Reinigungsmittel, Pflegemittel zur Behandlung von Oberflächen, beispielsweise von Möbeln, Fußböden, Kücheneinrichtungen, Glasscheiben und Fenstern sowie Bildschirmen, Bleichen, Toilettensteine, Entkalkungsmittel, Düngemittel, Baustoffe, Schimmelentferner, Desinfektionsmittel, Produkte für die Auto- bzw. Fahrzeugpflege und dergleichen mehr.

Die folgenden Beispiele dienen der Verdeutlichung der Erfindung, ohne sie in irgendeiner Weise zu beschränken.

### BEISPIELE

Gaschromatographische Analysen wurden nach der folgenden Methode durchgeführt:
Säule: DB WAX 30 m x 0,32 mm;
FD 0,25 µm;
Injektortemperatur: 200 °C; Detektortemperatur 280 °C;
Temperaturprogramm: Anfangstemp.: 50 °C, mit 3 °C/min auf 170 °C,
   mit 20 °C/min auf 230 °C, 7 Min isotherm;
Retentionszeiten:
   Isovaleraldehyd t_{R} = 3,7 min
   cis-Dihydrorosenoxid t_{R} = 8,4 min
   trans-Dihydrorosenoxid t_{R} = 9,6 min
   4,4-Dimethyl-2-isobutyl-1,3-dioxan t_{R} = 11,9 min

Konzentrationen der erhaltenen Rohprodukte (Gew.-%) wurden GC-analytisch mit internem Standard ermittelt.

### Beispiel 1 (erfindungsgemäß):

Ein Gemisch aus Isovaleraldehyd (112,5 g, 1,31 mol), Isoprenol (125 g, 1,45 mol) und 12,5 g Wasser wurde in Gegenwart von 50 g des stark sauren Kationenaustauschers Amberlyst ® 131 umgesetzt, wie in Beispiel 1 der WO2011/154330 beschrieben.

Das erhalten Rohprodukt hatte die folgende Zusammensetzung:
19,5 GC-Flächen-% trans-Pyranol (I)
56,1 GC-Flächen-% cis-Pyranol (I)
9,0 GC-Flächen-% Dihydropyran-Isomere 1-3
8,2 GC-Flächen-% 1,3 Dioxan
0,7 GC-Flächen-% Isovaleraldehyd
1,3 GC-Flächen-% Isoprenol
0,5 GC-Flächen-% Acetal
8,7 % Wasser (Karl-Fischer-Methode)

Das Rohprodukt wurde einer destillativen Auftrennung in einer Anordnung aus einer konventionellen Destillationskolonne und einer Trennwandkolonne unterzogen. Die Laborapparatur bestand aus zwei Laborkolonnen. Die Trennwirkung der ersten Kolonne entspricht etwa 15 theoretischen Böden. Für die Trennung der beiden Phasen des Kopfkondensates war ein Glas-Phasenscheider eingebaut. Die untere wässrige Phase wurde standgeregelt herausgefahren. Die obere organische Phase wurde mithilfe eines Rücklaufteilers in einem festen Verhältnis aufgeteilt, wobei ein Teil als Kopfprodukt abgetrennt und der andere Teil wurde oben auf die Kolonne zurückgeführt wurde. Der Zulauf zur Kolonne erfolgte zwischen den beiden Kolonnenschüssen. Der Zulaufstrom wurde mit Raumtemperatur zugefahren. Der Mengenstrom betrug 1000 g/h.

Dem Rohprodukt wurden 10 Gew.-ppm Schwefelsäure als 1 %ige Lösung in Wasser zugegeben.

Die Kolonne wurde bei 50 mbar Kopfdruck und einer Rücklaufmenge von 360 g/h betrieben. Dabei stellte sich ein Druckverlust von ca. 3,1 mbar ein. Am Kopf der Kolonne wurde eine Temperatur von 70 °C und im Sumpf eine Temperatur von 131 °C gemessen. Die Sumpfabzugsmenge wurde fest auf 776 g/h eingestellt. Die Kopfabzugsmenge betrug 131 g/h.

Die gewonnenen Fraktionen wurden gaschromatographisch mit Hilfe eines Standard-GCs analysiert. Gaschromatographische Analysen wurden nach der folgenden Methode durchgeführt:
Säule: DB WAX 30 m x 0,32mm; FD 0,25µm; Inj. 200 °C, Det. 280 °C; 50°C, 3°/' auf 170 °C - 20°/' auf 230 °C - 7 Min isoth., t_{R} = min; t_{R} (Isovaleraldehyd): 3,8; t_{R} (Dihydropyran-Isomere): 10,1; 12,0; 12,4; t_{R} (Isoprenol): 10,7; t_{R} (1,3-Dioxan): 12,2; t_{R} (Acetal): 24,8; t_{R} (trans-Pyranol): 28,5; t_{R} (cis-Pyranol): 30,0; Konzentrationen der erhaltenen Rohprodukte (Gew.-%) wurden GC-analytisch mittels internem Standard ermittelt.

Der aus dem Phasenscheider am Kopf der Kolonne abgezogene Kopfstrom enthielt:
1,2 % Wasser (Karl-Fischer-Methode)
3,8 GC-Flächen-% Isovaleraldehyd
44,3 GC-Flächen-% Dihydropyran-Isomere 1-3
8,9 GC-Flächen-% Isoprenol
38,9 GC-Flächen-% 1,3 Dioxan

Im Sumpfabzug wurden
0,04 GC-Flächen-% Isoprenol
2,37 GC-Flächen-% Dihydropyran-Isomere
2,1 GC-Flächen-% 1,3 Dioxan
23,5 GC-Flächen-% trans-Pyranol
67,4 GC-Flächen-% cis-Pyranol
gefunden.

Die Destillationsausbeute bezüglich cis- und trans-Pyranol betrug 100 %.

Die zweite Laborkolonne wurde als Trennwandkolonne aufgebaut. Die Trennleistung im Trennwand-bereich betrug ca. 32 theoretische Stufen. Die Gesamtzahl der theoretischen Stufen inklusive des Trennwandbereiches betrug ca. 50. Der Zulauf wurde auf Höhe der Mitte des Trennwandteils zugegeben. Als Zulaufstrom wurde das Gemisch aus dem Sumpfablauf der ersten Kolonne verwendet. Der Zulaufmengenstrom betrug 302,4 g/h. Die Kolonne wurde bei 10 mbar Kopfdruck und einem Rücklauf von 400 g/h betrieben. Am Kopf der Kolonne wurde eine Temperatur von 72°C und im Sumpf eine Temperatur von 124 °C (± 0,5 K) gemessen. Der Sumpfabzug wurde - auf 14 g/h (± 1 g/h) und die Destillatabnahme auf 26 g/h (± 1 g/h) eingestellt. Das Rücklaufverhältnis betrug somit etwa 15:1. Die Flüssigkeit wurde oberhalb der Trennwand in einem Verhältnis von 1 : 2 (Zulauf- : Entnahmeteil) aufgeteilt. Auf der der Zugabeseite gegenüberliegenden Seite der Trennwand wurde auf gleicher Höhe wie der Zulaufstrom ein flüssiger Seitenabzug entnommen. Der Mengenstrom war fest auf 261 g/h eingestellt.

Das am Seitenabzug gewonnene Reinprodukt enthielt:
24,6 GC-Flächen-% trans-Pyranol und
74,7 GC-Flächen-% cis-Pyranol

Olfaktorische Bewertung des Reinprodukts:
Riechstreifentest 30 min: entspricht der gewünschten Spezifikation
Gasraumtest: entspricht der gewünschten Spezifikation

Die Destillationsausbeute bezüglich cis- und trans-Pyranol betrug ca. 97,5 %.

### Beispiel 2 (Vergleich):

Es wurde wie in Beispiel 1 verfahren, wobei dem zur Destillation eingesetzten Rohprodukt keine Schwefelsäure zugesetzt wurde.

Das am Seitenabzug der Trennwandkolonne gewonnene Reinprodukt enthielt:
25,5 GC-Flächen-% trans-Pyranol und
73,6 GC-Flächen-% cis-Pyranol

Olfaktorische Bewertung des Reinprodukts:
Riechstreifentest 30 min: entspricht nicht der Spezifikation
Gasraumtest: entspricht nicht der Spezifikation

Die Destillationsausbeute bezüglich cis- und trans-Pyranol betrug ca. 98,0 %.

## Patentansprüche

1. Verfahren zur Herstellung von 2-substituierten 4-Hydroxy-4-methyl-tetrahydropyranen der allgemeinen Formel (I) 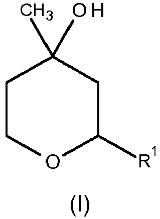 worin
R¹ für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
bei dem man
a) 3-Methylbut-3-en-1-ol der Formel (IV) 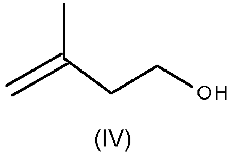 mit einem Aldehyd der Formel (V)
R¹-CHO (V)
worin
R¹ für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
in Gegenwart eines sauren Katalysators umsetzt, wobei ein Reaktionsgemisch erhalten wird, das wenigstens ein 2-substituiertes 4-Hydroxy-4-methyl-tetrahydropyran der allgemeinen Formel (I) enthält,
b) das Reaktionsgemisch aus Schritt a) einer destillativen Auftrennung unter Erhalt wenigstens einer an den 2-substituierten 4-Hydroxy-4-methyl-tetrahydropyranen der allgemeinen Formel (I) angereicherten Fraktion unterzieht,
wobei man das Reaktionsgemisch aus Schritt a) vor dem Einsatz in Schritt b) und/oder während des Einsatzes in Schritt b) mit einem sauren Ionenaustauscher in Kontakt bringt und/oder mit einer starken Säure versetzt.

2. Verfahren nach Anspruch 1, wobei man das Reaktionsgemisch aus Schritt a) während des Einsatzes in Schritt b) mit einem sauren Ionenaustauscher in Kontakt bringt und/oder mit einer starken Säure versetzt.

3. Verfahren nach Anspruch 1, wobei man eine bei der destillativen Auftrennung in Schritt b) erhaltene Fraktion mit einer starken Säure versetzt.

4. Verfahren nach einem der vorhergehenden Ansprüche, wobei man den Sumpf der destillativen Auftrennung in Schritt b) oder bei einer mehrstufigen Destillation den Sumpf der ersten Destillationsstufe der destillativen Auftrennung in Schritt b) mit einer starken Säure zu versetzen.

5. Verfahren nach einem der vorhergehenden, wobei man eine starke Säure einsetzt, die ausgewählt ist unter Schwefelsäure, Salzsäure, Methansulfonsäure und p-T oluolsulfonsäure.

6. Verfahren nach einem der vorhergehenden Ansprüche, wobei in Schritt a) ein Reaktionsgemisch erhalten wird, das
- wenigstens ein 2-substituiertes 4-Hydroxy-4-methyl-tetrahydropyran der allgemeinen Formel (I),
- wenigstens eine Dioxanverbindung (II) 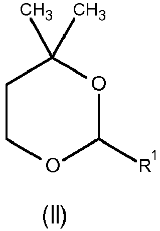
- und wenigstens eine der Verbindungen (III.1), (III.2) oder (III.3) 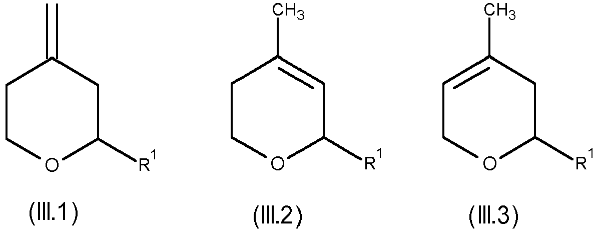 enthält, worin
R¹ für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht.

7. Verfahren nach Anspruch 6, wobei in Schritt a) ein Reaktionsgemisch erhalten wird, das zusätzlich wenigstens eine Verbindung enthält, die ausgewählt ist unter:
- 3-Methylbut-3-en-1-olen der Formel (IV)
- Aldehyden der Formel (V)
- Acetalen der allgemeinen Formel (VI) 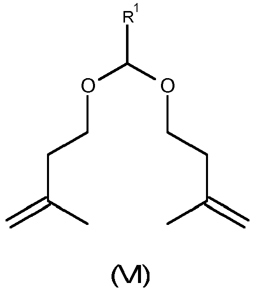 worin
R¹ für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht.

8. Verfahren nach einem der vorhergehenden Ansprüche, wobei der Rest R¹ für Isobutyl oder Phenyl steht.

9. Verfahren nach einem der vorhergehenden Ansprüche, wobei zur Auftrennung in Schritt b) eine Vorrichtung eingesetzt wird, die
- eine Zulaufsäule mit oberhalb der Zulaufstelle gelegenem Verstärkungsteil und unterhalb der Zulaufstelle gelegenem Abtriebsteil,
- eine mit dem oberen Ende des Verstärkungsteils kommunizierende obere Vereinigungssäule und eine mit dem unteren Ende des Abtriebsteils kommunizierende untere Vereinigungssäule, und
- eine mit der oberen Vereinigungssäule und der unteren Vereinigungssäule kommunizierende Abzugssäule
umfasst.

10. Verfahren nach einem der vorhergehenden Ansprüche, wobei die destillative Auftrennung in Schritt b) in einer Anordnung von Destillationskolonnen erfolgt, die eine Trennwandkolonne oder eine Zusammenschaltung von mindestens zwei thermisch gekoppelten konventionellen Destillationskolonnen umfasst.

11. Verfahren nach einem der vorhergehenden Ansprüche, wobei zur destillativen Auftrennung des Reaktionsprodukts aus Schritt a) eine Anordnung von Destillationskolonnen eingesetzt wird, die eine vorgeschaltete konventionelle Destillationskolonne und eine nachgeschaltete Trennwandkolonne oder eine nachgeschaltete Zusammenschaltung von zwei thermisch gekoppelten konventionellen Destillationskolonnen umfasst.

12. Verfahren nach einem der vorhergehenden Ansprüche, wobei zur destillativen Auftrennung in Schritt b)
b1) das Reaktionsgemisch aus Schritt a) zunächst einer Auftrennung in einer konventionellen Destillationskolonne unterzogen wird, wobei ein erstes Kopfprodukt erhalten wird, das an der Dioxanverbindung (II) und den Verbindungen (III.1), (III.2) und (III.3) und angereichert ist und im Wesentlichen keine Verbindungen der allgemeinen Formel (I) enthält, und ein erstes Sumpfprodukt erhalten wird, das an der Dioxanverbindung (II) und den Verbindungen (III.1), (III.2) und (III.3) abgereichert ist und die Hauptmenge der Verbindungen der allgemeinen Formel (I) enthält,
b2) das erste Sumpfprodukt aus Schritt b1) einer Auftrennung in einer Trennwandkolonne oder in einer Zusammenschaltung von zwei thermisch gekoppelten konventionellen Destillationskolonnen unterzogen wird, wobei ein zweites Kopfprodukt erhalten wird, das die nicht im ersten Kopfprodukt enthaltenen Verbindungen (II), (III.1), (III.2), (III.3) und sowie gegebenenfalls geringe Mengen der Verbindungen der allgemeinen Formel (I) enthält, ein Seitenstrom erhalten wird, der im Wesentlichen aus Verbindung der allgemeinen Formel (I) besteht und ein zweites Sumpfprodukt erhalten wird, das die Verbindungen der allgemeinen Formel (I) enthält, die nicht im Kopfprodukt und nicht im Seitenstrom enthalten sind.

13. Verfahren nach einem der vorhergehenden Ansprüche, bei dem man das mit Säure behandelte Reaktionsgemisch aus Schritt a
- einer ersten destillativen Auftrennung unterzieht, wobei ein erstes Kopfprodukt erhalten wird, das an der Dioxanverbindung (II) und den Verbindungen (III.1), (III.2) und (III.3) und angereichert ist und im Wesentlichen keine Verbindungen der allgemeinen Formel (I) enthält, und ein erstes Sumpfprodukt erhalten wird, das an der Dioxanverbindung (II) und den Verbindungen (III.1), (III.2) und (III.3) abgereichert ist und die Hauptmenge der Verbindungen der allgemeinen Formel (I) enthält,
- das erste Sumpfprodukt mit wenigstens einer Base versetzt oder einer einfachen Destillation unterzieht, wobei die Hauptmenge des ersten Sumpfprodukts verdampft und anschließend kondensiert wird,
- das mit Base versetzte erste Sumpfprodukt oder das Kondensat einer weiteren destillativen Auftrennung unterzogen wird.

14. Verfahren nach einem der vorhergehenden Ansprüche zur Herstellung von 2-Isobutyl-4-hydroxy-4-methyl-tetrahydropyran.

## Claims

1. A process for preparing 2-substituted 4-hydroxy-4-methyltetrahydropyrans of the general formula (I) 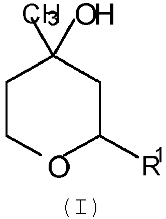 where
R¹ is straight-chain or branched C₁-C₁₂-alkyl, straight-chain or branched C₂-C₁₂-alkenyl, unsubstituted or C₁-C₁₂-alkyl- and/or C₁-C₁₂-alkoxy-substituted cycloalkyl having in total 3 to 20 carbon atoms or unsubstituted or C₁-C₁₂-alkyl- and/or C₁-C₁₂-alkoxy-substituted aryl having in total 6 to 20 carbon atoms,
wherein
a) 3-methylbut-3-en-1-ol of the formula (IV) 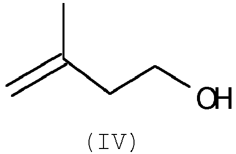 is reacted with an aldehyde of the formula (V)
R¹-CHO (V)
where
R¹ is straight-chain or branched C₁-C₁₂-alkyl, straight-chain or branched C₂-C₁₂-alkenyl, unsubstituted or C₁-C₁₂-alkyl- and/or C₁-C₁₂-alkoxy-substituted cycloalkyl having in total 3 to 20 carbon atoms or unsubstituted or C₁-C₁₂-alkyl- and/or C₁-C₁₂-alkoxy-substituted aryl having in total 6 to 20 carbon atoms,
in the presence of an acidic catalyst, wherein a reaction mixture is obtained comprising at least one 2-substituted 4-hydroxy-4-methyltetrahydropyran of the general formula (I),
b) the reaction mixture from step a) is subjected to a distillative separation to give at least one fraction enriched in the 2-substituted 4-hydroxy-4-methyltetrahydropyrans of the general formula (I) ,
wherein the reaction mixture from step a), prior to use in step b) and/or during use in step b), is brought into contact with an acidic ion exchanger and/or admixed with a strong acid.

2. The process according to Claim 1, wherein the reaction mixture from step a) during use in step b) is brought into contact with an acidic ion exchanger and/or admixed with a strong acid.

3. The process according to Claim 1, wherein a fraction obtained in the distillative separation in step b) is admixed with a strong acid.

4. The process according to any of the preceding claims, wherein a strong acid is added to the bottoms of the distillative separation in step b) or, in the case of a multi-stage distillation, to the bottoms of the first distillation stage of the distillative separation in step b).

5. The process according to any of the preceding claims, wherein a strong acid is used which is selected from sulfuric acid, hydrochloric acid, methanesulfonic acid and p-toluenesulfonic acid.

6. The process according to any of the preceding claims, wherein in step a) a reaction mixture is obtained which comprises
- at least one 2-substituted 4-hydroxy-4-methyltetrahydropyran of the general formula (I),
- at least one dioxane compound (II) 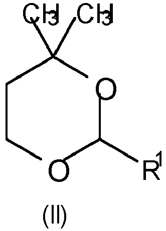
- and at least one of the compounds (III.1), (III.2) or (III.3) 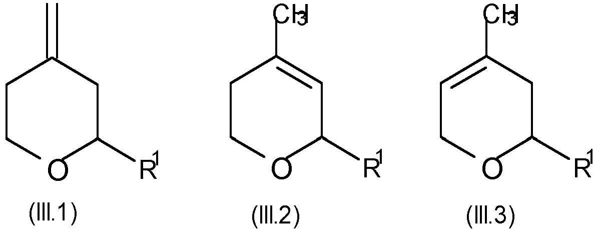 where
R¹ is straight-chain or branched C₁-C₁₂-alkyl, straight-chain or branched C₂-C₁₂-alkenyl, unsubstituted or C₁-C₁₂-alkyl- and/or C₁-C₁₂-alkoxy-substituted cycloalkyl having in total 3 to 20 carbon atoms or unsubstituted or C₁-C₁₂-alkyl- and/or C₁-C₁₂-alkoxy-substituted aryl having in total 6 to 20 carbon atoms.

7. The process according to Claim 6, wherein in step a) a reaction mixture is obtained which additionally comprises at least one compound selected from:
- 3-methylbut-3-en-1-ols of the formula (IV)
- aldehydes of the formula (V)
- acetals of the general formula (VI) 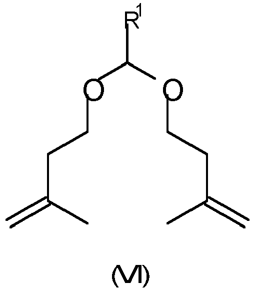 where
R¹ is straight-chain or branched C₁-C₁₂-alkyl, straight-chain or branched C₂-C₁₂-alkenyl, unsubstituted or C₁-C₁₂-alkyl- and/or C₁-C₁₂-alkoxy-substituted cycloalkyl having in total 3 to 20 carbon atoms or unsubstituted or C₁-C₁₂-alkyl- and/or C₁-C₁₂-alkoxy-substituted aryl having in total 6 to 20 carbon atoms.

8. The process according to any of the preceding claims, wherein the radical R¹ is isobutyl or phenyl.

9. The process according to any of the preceding claims, wherein, for the separation in step b), a device is used which comprises
- a feed column with rectification section situated above the feed point and stripping section positioned below the feed point,
- an upper combining column communicating with the upper end of the rectification section and a lower combining column communicating with the lower end of the stripping section, and
- a discharge column communicating with the upper combining column and the lower combining column.

10. The process according to any of the preceding claims, wherein the distillative separation in step b) takes place in an arrangement of distillation columns which comprises a dividing-wall column or an interconnection of at least two thermally coupled conventional distillation columns.

11. The process according to any of the preceding claims, wherein, for the distillative separation of the reaction product from step a), an arrangement of distillation columns is used which comprises an upstream conventional distillation column and a downstream dividing-wall column or a downstream interconnection of two thermally coupled conventional distillation columns.

12. The process according to any of the preceding claims, wherein, for the distillative separation in step b)
b1) the reaction mixture from step a) is initially subjected to a separation in a conventional distillation column, where a first top product is obtained which is enriched in the dioxane compound (II) and the compounds (III.1), (III.2) and (III.3) and comprises essentially no compounds of the general formula (I), and a first bottom product is obtained which is depleted in the dioxane compound (II) and the compounds (III.1), (III.2) and (III.3) and comprises the majority of the compounds of the general formula (I) ,
b2) the first bottom product from step b1) is subjected to a separation in a dividing-wall column or in an interconnection of two thermally coupled conventional distillation columns, where a second top product is obtained which comprises the compounds (II), (III.1), (III.2), (III.3) not present in the first top product, as well as optionally small amounts of the compounds of the general formula (I), a side stream is obtained which consists essentially of compound of the general formula (I), and a second bottom product is obtained which comprises the compounds of the general formula (I) which are not present in the top product and not present in the side stream.

13. The process according to any of the preceding claims, in which the reaction mixture treated with acid from step a)
- is subjected to a first distillative separation, where a first top product is obtained which is enriched in the dioxane compound (II) and the compounds (III.1), (III.2) and (III.3) and comprises essentially no compounds of the general formula (I), and a first bottom product is obtained which is depleted in the dioxane compound (II) and the compounds (III.1), (III.2) and (III.3) and comprises the majority of the compounds of the general formula (I),
- the first bottom product is admixed with at least one base or subjected to a simple distillation, where the majority of the first bottom product is evaporated and then condensed,
- the first bottom product admixed with base or the condensate is subjected to a further distillative separation.

14. The process according to any of the preceding claims for preparing 2-isobutyl-4-hydroxy-4-methyltetrahydropyran.

## Revendications

1. Procédé pour la préparation de 4-hydroxy-4-méthyl-tétrahydropyrannes substitués en position 2 de formule générale (I) 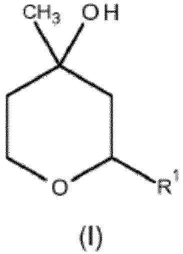 dans laquelle
R¹ représente C₁₋₁₂-alkyle linéaire ou ramifié, C₂-₁₂-alcényle linéaire ou ramifié, cycloalkyle comportant au total 3 à 20 atomes de carbone non substitué ou substitué par C₁₋₁₂-alkyle et/ou C₁₋₁₂-alcoxy ou aryle comportant au total 6 à 20 atomes de carbone non substitué ou substitué par C₁₋₁₂alkyle et/ou C₁₋₁₂-alcoxy,
dans lequel
a) on transforme du 3-méthylbut-3-én-1-ol de formule (IV) 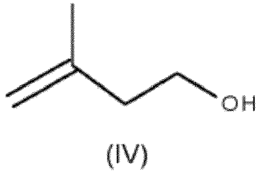 avec un aldéhyde de formule (V)
**R¹-CHO** **(V)**
dans laquelle
R¹ représente C₁-₁₂-alkyle linéaire ou ramifié, C₂-₁₂-alcényle linéaire ou ramifié, cycloalkyle comportant au total 3 à 20 atomes de carbone non substitué ou substitué par C₁-₁₂-alkyle et/ou C₁-₁₂-alcoxy ou aryle comportant au total 6 à 20 atomes de carbone non substitué ou substitué par C₁₋₁₂-alkyle et/ou C₁₋₁₂-alcoxy,
en présence d'un catalyseur acide, un mélange réactionnel étant obtenu, qui contient au moins un 4-hydroxy-4-méthyl-tétrahydropyranne substitué en position 2 de formule générale (I),
b) on soumet le mélange réactionnel de l'étape a) à une séparation par distillation avec obtention d'au moins une fraction enrichie en 4-hydroxy-4-méthyl-tétrahydropyrannes substitués en position 2 de formule générale (I),
le mélange réactionnel de l'étape a) étant mis en contact avec un échangeur d'ions acide et/ou mélangé avec un acide fort avant l'utilisation dans l'étape b) et/ou pendant l'utilisation dans l'étape b).

2. Procédé selon la revendication 1, le mélange réactionnel de l'étape a) étant mis en contact avec un échangeur d'ions acide et/ou mélangé avec un acide fort pendant l'utilisation dans l'étape b).

3. Procédé selon la revendication 1, une fraction obtenue lors de la séparation par distillation dans l'étape b) étant mélangée avec un acide fort.

4. Procédé selon l'une quelconque des revendications précédentes, le fond de la séparation par distillation dans l'étape b) ou, lors d'une distillation en plusieurs étapes, le fond de la première étape de distillation de la séparation par distillation dans l'étape b) étant mélangé avec un acide fort.

5. Procédé selon l'une quelconque des revendications précédentes, un acide fort étant utilisé, qui est choisi parmi l'acide sulfurique, l'acide chlorhydrique, l'acide méthanesulfonique et d'acide p-toluènesulfonique.

6. Procédé selon l'une quelconque des revendications précédentes, où dans l'étape a) un mélange réactionnel est obtenu, qui contient
- au moins un 4-hydroxy-4-méthyl-tétrahydropyranne substitué en position 2 de formule générale (I),
- au moins un composé de dioxanne (II) 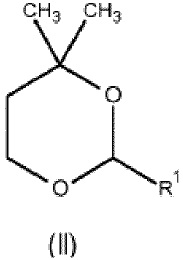
- et au moins un des composés (III.1), (III.2) ou (III.3) 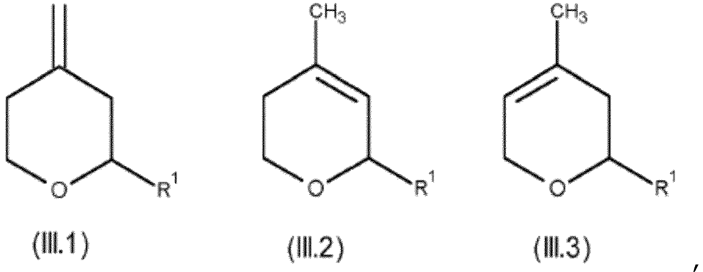 dans lesquels
R¹ représente C₁₋₁₂-alkyle linéaire ou ramifié, C₂₋₁₂-alcényle linéaire ou ramifié, cycloalkyle comportant au total 3 à 20 atomes de carbone non substitué ou substitué par C₁₋₁₂-alkyle et/ou C₁₋₁₂-alcoxy ou aryle comportant au total 6 à 20 atomes de carbone non substitué ou substitué par C₁₋₁₂-alkyle et/ou C₁₋₁₂-alcoxy.

7. Procédé selon la revendication 6, où dans l'étape a), un mélange réactionnel est obtenu, qui contient de plus au moins un composé qui est choisi parmi :
- des 3-méthylbut-3-én-1-ols de formule (IV)
- des aldéhydes de formule (V)
- des acétals de formule générale (VI) 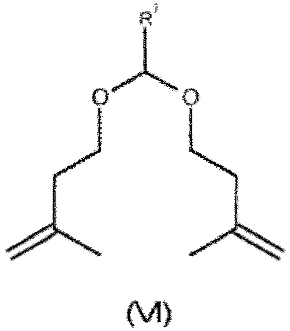 dans laquelle
R¹ représente C₁₋₁₂-alkyle linéaire ou ramifié, C₂₋₁₂-alcényle linéaire ou ramifié, cycloalkyle comportant au total 3 à 20 atomes de carbone non substitué ou substitué par C₁₋₁₂-alkyle et/ou C₁₋₁₂-alcoxy ou aryle comportant au total 6 à 20 atomes de carbone non substitué ou substitué par C₁₋₁₂-alkyle et/ou C₁₋₁₂-alcoxy.

8. Procédé selon l'une quelconque des revendications précédentes, le radical R¹ représentant isobutyle ou phényle.

9. Procédé selon l'une quelconque des revendications précédentes, où pour la séparation dans l'étape b) un dispositif est utilisé, qui comprend
- une colonne d'alimentation avec une zone de renforcement située au-dessus du site d'alimentation et une zone de rectification située au-dessous du site d'alimentation,
- une colonne de réunification supérieure communiquant avec l'extrémité supérieure de la zone de renforcement et une colonne de réunification inférieure communiquant avec l'extrémité inférieure de la zone de rectification, et
- une colonne de soutirage communiquant avec la colonne de réunification supérieure et la colonne de réunification inférieure.

10. Procédé selon l'une quelconque des revendications précédentes, la séparation par distillation dans l'étape b) étant réalisée dans un ensemble de colonnes de distillation qui comprend une colonne à paroi de séparation ou une interconnexion d'au moins deux colonnes de distillation conventionnelles couplées thermiquement.

11. Procédé selon l'une quelconque des revendications précédentes, où pour la séparation par distillation du produit de réaction de l'étape a), un ensemble de colonnes de distillation est utilisé, qui comprend une colonne de distillation conventionnelle montée en amont et une colonne à paroi de séparation montée en aval ou une interconnexion, montée en aval, de deux colonnes de distillation conventionnelles couplées thermiquement.

12. Procédé selon l'une quelconque des revendications précédentes, où pour la séparation par distillation dans l'étape b)
b1) le mélange réactionnel de l'étape a) est soumis tout d'abord à une séparation dans une colonne de distillation conventionnelle, un premier produit de tête étant obtenu, qui est enrichi en composé de type dioxanne (II) et en composés (III.1), (III.2) et (III.3) et ne contenant essentiellement aucun composé de formule générale (I), et un premier produit de fond étant obtenu, qui est appauvri en composé de type dioxanne (II) et en composés (III.1), (III.2) et (III.3) et contenant la quantité principale des composés de formule générale (I),
b2) le premier produit de fond de l'étape b1) est soumis à une séparation dans une colonne à paroi de séparation ou dans une interconnexion de deux colonnes de distillation conventionnelles couplées thermiquement, un deuxième produit de tête étant obtenu, qui contient les composés (II), (III.1), (III.2) et (III.3) qui ne sont pas contenus dans le premier produit de tête ainsi qu'éventuellement de faibles quantités de composés de formule générale (I), un flux latéral étant obtenu, qui est essentiellement constitué du composé de formule générale (I) et un deuxième produit de fond étant obtenu, qui contient les composés de formule générale (I) qui ne sont pas contenus dans le produit de tête et dans le flux latéral.

13. Procédé selon l'une quelconque des revendications précédentes, dans lequel le mélange réactionnel traité par de l'acide de l'étape a
- est soumis à une première séparation par distillation, un premier produit de tête étant obtenu, qui est enrichi en composé de type dioxanne (II) et en composés (III.1), (III.2) et (III.3) et ne contient essentiellement aucun composé de formule générale (I), et un premier produit de fond étant obtenu, qui est appauvri en composé de type dioxanne (II) et en composés (III.1), (III.2) et (III.3) et contient la quantité principale des composés de formule générale (I),
- le premier produit de fond est mélangé avec au moins une base ou est soumis à une distillation simple, la quantité principale du premier produit de fond étant évaporée et ensuite condensée,
- le premier produit de fond mélangé avec une base ou le condensat est soumis à une séparation supplémentaire par distillation.

14. Procédé selon l'une quelconque des revendications précédentes pour la préparation de 2-isobutyl-4-hydroxy-4-méthyl-tétrahydropyranne.
